# NEW EUROPEAN PATENT SPECIFICATION

(11) **EP 1 654 361 B2**
(45) Date of publication and mention of the opposition decision: **18.01.2023**
(45) Mention of the grant of the patent: 18.04.2012
(21) Application number: 04820001.8
(22) Date of filing: 26.07.2004
(51) Int. Cl.: C12N 15/10

(54) **METHODS AND COMPOSITIONS FOR PREPARING RNA FROM A FIXED SAMPLE**
VERFAHREN UND ZUSAMMENSETZUNGEN ZUR HERSTELLUNG VON RNA AUS EINER FIXIERTEN PROBE
PROCEDES ET COMPOSITIONS DE PREPARATION D'UNE ARN A PARTIR D'UN ECHANTILLON FIXE

(30) Priority: 25.07.2003 US 490325 P
(43) Date of publication of application: 10.05.2006
(60) Divisional application: 10184549.3
(73) Proprietor: Life Technologies Corporation, Carlsbad, CA 92008 (US)
(72) Inventor: CONRAD, Richard, C., Austin, TX 78749-1740 (US); ZERINGER, Emily, Austin, TX 78749 (US)
(74) Representative: HGF
(86) International application number: PCT/US2004/024155
(87) International publication number: WO 2005/054466

(56) References cited:
- EP-A2- 0 389 063
- WO-A1-01/21830
- WO-A1-95/01359
- WO-A1-95/21849
- WO-A1-98/59076
- WO-A1-03/078662
- WO-A2-01/71732
- WO-A2-02/04620
- WO-A2-02/44339
- WO-A2-02/46463
- WO-A2-96/18731
- WO-A2-99/16781
- WO-A2-03/033722
- WO-A2-2004/033660
- US-A- 5 155 018
- US-B1- 6 218 531
- PALMIERI GIUSEPPE ET AL: "Detection of occult melanoma cells in paraffin-embedded histologically negative sentinel lymph nodes using a reverse transcriptase polymerase chain reaction assay" JOURNAL OF CLINICAL ONCOLOGY, vol. 19, no. 5, 1 March 2001 (2001-03-01), pages 1437-1443, XP002320654 ISSN: 0732-183X
- BOCK OLIVER ET AL: "One-step extraction of RNA from archival biopsies" ANALYTICAL BIOCHEMISTRY, vol. 295, no. 1, 1 August 2001 (2001-08-01), pages 116-117, XP002320655 ISSN: 0003-2697
- LEHMANN ULRICH ET AL: "Real-time PCR analysis of DNA and RNA extracted from formalin-fixed and paraffin-embedded biopsies" METHODS (ORLANDO), vol. 25, no. 4, December 2001 (2001-12), pages 409-418, XP002320656 ISSN: 1046-2023
- ANONYMOUS: "Thrombin Kits (TB 188 8/98)" NOVAGEN, [Online] 1998, XP002320657 Retrieved from the Internet: URL:http://www.emdbiosciences.com/docs/doc s/PROT/tb188.pdf> [retrieved on 2005-03-10]
- MASUDA NORIKAZU ET AL: "Analysis of chemical modification of RNA from formalin-fixed samples and optimization of molecular biology applications for such samples" NUCLEIC ACIDS RESEARCH, vol. 27, no. 22, 15 November 1999 (1999-11-15), pages 4436-4443, XP002320835 ISSN: 0305-1048
- Krafft et al, (1997) "Optimization of the isolation and amplification of RNA from formalin- fixed, paraffin-embedded tissue: The armed forces institute of pathology experience and literature review", Molecular Diagnosis 2: 217-230
- Koopmans et al, (1993) "Optimization of extraction and PCR amplification of RNA extracts from paraffin-embedded tissue indifferent fixatives"; Journal of Virological Methods 43: 189-204
- Jackson et al, (1990) "Tissue extraction of DNA and RNA analysis by the polymerase chain reaction", J Clin Pathol 43:499-504
- von Weizsacker et al, (1991) "A simple and rapid method for the detection of RNA in formalin-fixed, paraffin-embedded tissues by PCR amplification", Biochem Biophys Res Common 174: 176-180
- Finke et al, (1993) "An improved strategy and a useful housekeeping gene for RNA analysis from formalin-fixed, paraffin-embedded tissues by PCR" Biotechniques 14(3): 448-453
- Chung et al, (2002), Detection and differentiation of North American and European genotypes of porcine reproductive and respiratory syndrome virus in formalin-fixed, paraffin-embedded tissues by multiplex reverse transcription-nested polymerase chain reaction": J Vet Diagn Invest 14: 56-60
- Jung et al, (March 2003) "Differentiation between porcine epidemic diarrhea virus and transmissible gastroenteritis virus in formalin-fixed paraffin-embedded tissues by multiplex RT-nested PCR and comparison with in situ hybridisation"; J Virol Methods 108: 41-47
- Specht et al, (2001) "Quantitative gene expression analysis in microdissected archival formalin-fixed and paraffin-embedded tumor tissue"; American Journal of Pathology 158(2): 419-429
- Sorg et al, (1995) "Detection of borna disease virus RNA in formalin fixed, paraffin- embedded brain tissues by nested PCR"; Journal of Clinical Microbiology 33(4): 821-823
- Bresters et al, (1992) "Detection of hepatitis C viral RNA sequences in fresh and paraffin-embedded liver biopsy specimens of non-A, non-B hepatitis patients": J Hepatol 15: 391-395
- Choi et al, (January 2004) "Development of nested RT-PCR for the detection of swine hepatitis E virus in formalin-fixed paraffin-embedded tissues and comparison with in situ hybridization" J Viral Methods 115(1) 67-71
- Rupp et al, (1988), Purification and analysis of RNA from paraffin-embedded tissues". Biotechniques 6(1) 56-60
- Mizuno el al, (1998) "RNA from decades-old archival tissue blocks for retrospective studies?, Diagnostic Molecular Pathology 7(4): 202-208
- Palmieri et al, (1999) "Polymerase chain reaction-based detection of circulating melanoma cells as an effective marker of tumor progression" J Clin Oncol. 17: 304-311
- Stanta et al, (1991) "RNA extracted from paraffin-embedded human tissues is amenable to analysis by PCR amplification" Biotechniques 11 304. 306. 308
- Fisher et al, (1988) "Activity of proteinase K and RNase in guanidinium thiocyanate" FASEB J 2: A1126 (abstr.)
- Tyrell et al, (1995) "Detection of specific mRNAs in routinely processed dermatophathology specimes" Am J Dermatopathol 17:476-483
- Godfrey et al, (2000) "Quantitative mRNA expression analysis from formalin-fixed, paraffin-embedded tissues using 5'nuclease quantitative reverse transcription-polymerase chain reaction" J. Mol Diagn 2: 84-91
- Masuda et al, (1999} "Analysis of chemical modification of RNA from formalin-fixed samples and optimization of molecular biology applications for such samples"; Nucleic Acid Research 27(22): 4436-4443
- Bock et al, (2001) Analytical Biochemistry 295. 116-117
- Lehmann et al. (2001) Real-time PCR analysis of DNA and RNA extracted from formalin-fixed and paraffin-embedded biopsies"; Methods 25; 409-418
- Srinivasan et al, (2002) "Effect of fixatives and tissue processing on the content and integrity of nucleic acid", Am J Pathology 161(6):1961-1971
- Macabeo-Ong et al, (2001) "Effect of duration of fixation on quantitative reverse transcription polymerase chain reaction analyses" Mod Pathol 15(9) 979-987
- Proteinase K product description from Roche (January 2003)
- Proteinase K product description from Invitrogen (2001)
- RNeasy Mini Handbook from Qiagen (June 2001)
- Protocol Total RNA isolation from microdissected formalin-fixed tissues from Qiagen, April 2003
- High Pure RNA Paraffin Kit - Instruction Manual from Roche,(August 2003)
- Gribanov et al, (1997) Bioorganicheskaya Khimiya 23(9): 763-765
- Translation of D35
- Chomczynski and Sacchi (1987) Anal Biochem Apr; 162(1): 156-9
- Palmieri et al (2001) J Clin Oncol. Mar 1; 19(5): 1437-43
- Okello et al (2010) Anal Biochem May; 400(1): 110-7
- Conrad Agreement
- Zeringer Agreement
- Inventor Declarations including Annex 1
- Pages 93 to 95 from inventor's lab book
- Product information on the Recover All? Total Nucleic Acid Isolation Kit for isolating RNA from FFPE samples
- Evidence that a German Partnerschaftsgesellschaft is a legal body entitled to file an opposition

### SEQUENCE LISTING

<110> CONRAD, RICHARD
   ZERINGER, EMILY
   <120> METHODS AND COMPOSITIONS FOR PREPARING RNA FROM A FIXED SAMPLE
<130> AMBI : 088WO
<140> UNKNOWN
   <141> 2004-07-26
<150> 60/490,325 <151> 2003-07-25
<160> 12
<170> PatentIn Ver. 2.1
<210> 1
   <211> 20
   <212> DNA
   <213> Artificial Sequence
<220>
   <223> Description of Artificial Sequence: Synthetic Primer
<400> 1
   tgccgatgcc cccatgtttg 20
<210> 2
   <211> 18
   <212> DNA
   <213> Artificial Sequence
<220>
   <223> Description of Artificial Sequence: Synthetic Primer
<400> 2
   tcatcatctc cgcccctt 18
<210> 3
   <211> 20
   <212> DNA
   <213> Artificial Sequence
<220>
   <223> Description of Artificial Sequence: Synthetic Primer
<400> 3
   tctcgtggtt cacacccatc 20
<210> 4
   <211> 21
   <212> DNA
   <213> Artificial Sequence
<220>
   <223> Description of Artificial Sequence: Synthetic Primer
<400> 4
   ccttccgtga gtgcgaggca c 21
<210> 5
   <211> 26
   <212> DNA
   <213> Artificial Sequence
<220>
   <223> Description of Artificial Sequence: Synthetic Primer
<400> 5
   cagcatcaaa ggcttttata caagtg 26
<210> 6
   <211> 18
   <212> DNA
   <213> Artificial Sequence
<220>
   <223> Description of Artificial Sequence: Synthetic Primer
<400> 6
   tgccatcgac ctcatcca 18
<210> 7
   <211> 23
   <212> DNA
   <213> Artificial Sequence
<220>
   <223> Description of Artificial Sequence: Synthetic Primer
<400> 7
   cctgagggac cctaccgcat agc 23
<210> 8
   <211> 21
   <212> DNA
   <213> Artificial Sequence
<220>
   <223> Description of Artificial Sequence: Synthetic Primer
<400> 8
   cctggatagc attccgaacc t 21
<210> 9
   <211> 22
   <212> DNA
   <213> Artificial Sequence
<220>
   <223> Description of Artificial Sequence: Synthetic Primer
<400> 9
   agcacatctc gaaggctaca ca 22
<210> 10
   <211> 23
   <212> DNA
   <213> Artificial Sequence
<220>
   <223> Description of Artificial Sequence: Synthetic Primer
<400> 10
   agcaagtcac ttttcaagcg gct 23
<210> 11
   <211> 24
   <212> DNA
   <213> Artificial Sequence
<220>
   <223> Description of Artificial Sequence: Synthetic Primer
<400> 11
   tcaaatggtc cattaagcaa acaa 24
<210> 12
   <211> 21
   <212> DNA
   <213> Artificial Sequence
<220>
   <223> Description of Artificial Sequence : Synthetic Primer
<400> 12
   gctgcaccta gcctcttgaa a 21

## Description

### 1. Field of the Invention

The present invention relates generally to the field of molecular biology. More particularly, it concerns methods and compositions for isolating RNA of high quality and yield from fixed tissue.

### 2. Description of Related Art

RNA is often isolated from fixed tissue however, due to the processes involved in fixing tissue, such as the use of formaldehyde, the RNA obtained is fragmented. For studies of an RNA sample to be meaningful, it is necessary that the integrity of the RNA be maintained. Thus, fragmented RNA may bias the interpreted levels of RNA analyzed, for example, for expression levels of specific genes.

The use of formaldehyde as a preservative for animal tissue has existed for over a century. It provides the benefits of maintaining the structure of the tissue by "hardening" it, as well as serving as antibiotic agent to keep the tissue from physically rotting. These dual actions result from its rapid chemical reaction with tissue molecules, primarily protein, rendering the tissue highly cross-linked. This provides structural rigidity and stops diffusion of larger molecules between and within cells. This effectively keeps the tissue from rotting, since it stops all metabolism in the tissue itself and in any microorganisms carried within it. With current methods available for the examination of genes and gene expression through the use of extracted RNA and DNA, archived samples such as zoological and clinical specimens provide a wealth of material from which retrospective studies could be performed. Unfortunately, the same reactions that preserve the tissue serve to render it recalcitrant to extraction of either RNA or DNA. Procedures to perform this function have been reported in the scientific literature. However, these procedures are limited in their ability to obtain RNA of very high quality or yield (as judged by yield from unfixed tissue of similar origin).

Formaldehyde fixes tissue by forming methylene crosslinks between nitrogen atoms in biological macromolecules. Most of these chemical adducts are in protein, and a small percentage also involve the base of nucleic acids. Nucleic acids are trapped in the fixed tissue both by the formation or highly-crosslinked protein "cages," as well as being involved in a limited number of the crosslinks themselves. The published approaches to retrieval of RNA from fixed tissue have primarily concentrated on removing all vestiges of protein through enzymatic methods, although some have also tried chemico-physical methods as well. The procedures using proteolytic degradation have relied primarily on proteinase K, a protease with little amino acid specificity that can function in the presence of moderately denaturing conditions. The most common form of this procedure is to use it in the presence of 2% SDS at elevated temperature (37°C - 65°C).

Many procedures have been reported in the literature that profess to enable the retrieval and analysis of RNA from tissue samples that have been fixed in formaldehyde (see Masuda *et al.,* 1999; Danenberg et al., U.S. Patents 6,248,535 and 6,428,963; Fang *et al.,* 2002; Abrahamsen *et al.,* 2003; Liu *et al.,* 2002; Van Deerlin *et al.,* 2002; Karsten *et al.,* 2002; Godfrey *et al.,* 2000; Coombs *et al.,* 1999; Koopmans *et al.,* 1993; Specht *et al.,* 2001). In most of these procedures the final analysis is performed by looking at PCR products from the extracted RNA which has been reverse-transcribed ("RT-PCR"). The regions amplified in these procedures (the "amplicon") are inevitably only a few hundred nucleotides long at the maximum, and if a variation of the procedure known as real-time or quantitative PCR is used, the amplicons are usually 100 nucleotides or less.

When these procedures were applied and the RNA extracted by electrophoresis analyzed, it was apparent that the RNA obtained was heavily fragmented, with an average size in the hundred-nucleotide range. Presumably these fragments are providing a template for RT-PCR analysis. This result could easily be accomplished by ensuring the removal of only fragmented RNA, where the trapping crosslinks are located further apart than the average size of the fragments obtained. Although some authors aver that the process of formalin-fixation in itself fragments the RNA (Krafft *et al.,* 1997), this has been contradicted by others (Masuda *et al,* 1999).

Extremely fragmented RNA from fixed tissue was also obtained in procedures using citrate and guanidinium (Bock *et al.,* 2001). In addition, this procedure used high concentrations of proteinase K, citrate and detergent, including a reductant such as β-mercaptoethanol, which enhanced the production of fragmented RNA. The drawback of this procedure is that the integrity of the RNA was not maintained (not full-length) nor did it provide RNA of high quality and yield. [insert page 3a]

Any process that tends to affect a particular subset of the RNA present in a cell can bias the interpreted levels of RNA. Obviously, a process that maximizes yield while maintaining as much integrity as possible is the most desirable procedure to isolate both RNA and DNA. Thus, new or improved methods are needed for maximizing the yield of RNA from a fixed tissue sample in addition to obtaining full-length and substantially full-length RNA of high quality.

### SUMMARY OF THE INVENTION

The present invention concerns methods and kits for obtaining RNA, from a biological sample that has been fixed as defined by the claims. The invention is effective for obtaining RNA by isolating, extracting, and enriching for RNA, including full-length and substantially full-length RNA, from a sample using a digestion buffer or solution that includes a polyanion, wherein the polyanion is a polycarboxylate, selected from the group consisting of sodium citrate, 1,4-cyclohexanedicarboxylic acid, 1,3,5-cyclohexanetricarboxylic acid, isocitric acid, and succinic acid, preferably sodium citrate.

It is thus contemplated that methods and compositions of the invention can be employed to obtain a better yield of RNA from a sample, but also to obtain a better yield with respect to full-length RNA from the sample. The invention, in some embodiments, allows for extraction of about or at least about 10%, 20%, 30%, 40%, 50%, 60%, 70%, 80%, 90% or any range therein of either RNA from the sample. Also, the invention allows, in some embodiments for about or at least about 10%, 20%, 30%, 40%, 50%, 60%, 70%, 80%, 90% or any range therein of extracted RNA from a sample to be full-length or substantially full-length. The term "substantially full-length" means that the RNA appears or is determined to be at least 350 nucleotides in length. It is also contemplated that methods and compositions of the invention allow for the extraction of RNA, wherein at least about 50%, 60, 70%, 80%, 90% or more of the extracted RNA is at least about 50%, 60%, 70%, 80%, 90% or more intact.

A "digestion buffer or solution" is understood to be a buffer or solution (buffers are a type of solution) that has one or more compounds or agents that break down or digest one or more substances in a biological sample, such as one or more components of a cell. In embodiments of the invention, the digestion buffer or solution can be used on whole cells to create a lysate, which refers to the contents released from a lysed cell.

The term "polyanion" is used according to its ordinary and plain meaning to refer to a chemical compound that has more than one negative charge associated with it, such as -2, -3, -4, or more.

Palmieri et al (J. Clin Oncol. 19: 1437-1443, 2001) described a similar method using a lysis buffer consisting of Proteinase K, 4 M guanidinium thiocyanate, sodium citrate, sarcosyl and mercaproethanol to extract RNA from paraffin-embedded tissue sections from the lysis solution by phenol/chloroform extraction followed by isopropanol precipitation. From the extracted RNA amplicons of 158, 125, 157, and 128 bp length could be amplified by RT-PCR.

US patent 6,218,531 discloses a method for isolating RNA wherein a lysate formed by adding a lysis buffer containing guanidine hydrochloride as a chaotropic salt to the sample, precipitating proteins and DNA followed by centrifugation and applying the clear supernatant to a mineral support. The method can be used for various sources of tissues and cells or organelles, but remains silent on its applicability to fixed tissues.

The digestion buffer comprises a polyanion that is a polycarboxylate, which refers to a chemical compound that has at least one carboxylate group (COO⁻). Polycarboxylates include sodium citrate, trans-aconitic acid, 1,2,4-butanetricarboxylic acid, 1,4-cyclohexanedicarboxylic acid, 1,2,3,4,5,6-cyclohexanehexacarboxylic acid, isocitric acid, tricarballylic acid, succinic acid, and/or glutaric acid. It will be understood that the acid form of a compound can be found as a polyanion in solution, and thus, reference to the acid form is commensurate with referring to the acid in its polyanion form. In certain embodiments, the digestion buffer contains sodium citrate (NaCitrate). It is contemplated that the digestion buffer may contain more than one polyanion compound, and may contain at least 1, 2, 3, 4 or more such compounds in the digestion buffer.

The concentration of the polyanion in the digestion buffer, in some embodiments, is between about 1 mM and about 100 mM or between about 5 mM and about 50 mM. The concentration of the polyanion is about, at least about, or at most about 1, 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, 50, 51, 52, 53, 54, 55, 56, 57, 58, 59, 60, 61, 62, 63, 64, 65, 66, 67, 68, 69, 70, 71, 72, 73, 74, 75, 76, 77, 78, 79, 80, 81, 82, 83, 84, 85, 86, 87, 88, 89, 90, 91, 92, 93, 94, 95, 96, 97, 98, 99, 100 or more mM, or any range therein, in the digestion buffer or as a final concentration with the sample.

Digestion buffers can be used to create a cell lysate when exposed to whole cells, tissue, or organs. A lysate results when a cell is lysed or its integrity disrupted. Components of a digestion buffer can include proteases, nucleases (particularly non-RNases), and/or other compounds that chemically or enzymatically disrupt components of a cell. A digestion buffer may include one or more of such components. In particular embodiments of the invention, the digestion buffer includes a protease (also referred to as a peptidase or proteinase), which is an enzyme that catalyzes the breakdown of peptide bonds (known as proteolysis). It is contemplated that the amount of protease in digestion buffers of the invention is an effective amount to achieve lysis of cells in a sample. In further embodiments, the protease is proteinase K, though the invention is not limited to this embodiment. The concentration of the protease can be about, at least about, or at most about 5, 10, 15, 20, 25, 30, 35, 40, 45, 50, 55, 60, 65, 70, 75, 80, 85, 90, 95, 100, 110, 120, 130, 140, 150, 160, 170, 180, 190, 200, 210, 220, 230, 240, 250, 260, 270, 280, 290, 300, 310, 320, 330, 340, 350, 360, 370, 380, 390, 400, 410, 420, 430, 440, 450, 460, 470, 480, 490, 500, 510, 520, 530, 540, 550, 560, 570, 580, 590, 600, 610, 620, 630, 640, 650, 660, 670, 680, 690, 700, 710, 720, 730, 740, 750, 760, 770, 780, 790, 800, 810, 820, 830, 840, 850, 860, 870, 880, 890, 900, 910, 920, 930, 940, 950, 960, 970, 980, 990, 1000 µg/ml or any range therein.

The digestion buffer of the invention can further include a salt, which is sodium in some embodiments of the invention. The concentration of sodium is between about, at least about, or at most about 1, 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, 50, 51, 52, 53, 54, 55, 56, 57, 58, 59, 60, 61, 62, 63, 64, 65, 66, 67, 68, 69, 70, 71, 72, 73, 74, 75, 76, 77, 78, 79, 80, 81, 82, 83, 84, 85, 86, 87, 88, 89, 90, 91, 92, 93, 94, 95, 96, 97, 98, 99, 100,110, 120, 130, 140, 150, 160, 170, 180, 190, 200, 210, 220, 230, 240, 250, 260, 270, 280, 290, 300, 310 320, 330, 340, 350, 360, 370, 380, 390, 400, 410, 420, 430, 440, 450, 460, 470, 480, 490, 500 mM or more, or any range therein. The sodium may be provided in the buffer as NaCl. In addition, it may be provided in the buffer in multiple ways, such as by adding more than one compound that includes sodium.

It is contemplated that the pH of the digestion buffer, or of the buffer component of the digestion buffer, or of the digestion buffer with the sample is between about 6.5 and 9.5, though it can be about, about at least, or about at most 6.5, 6.6, 6.7, 6.8, 6.9, 7.0, 7.1, 7.2, 7.3, 7.4, 7.5, 7.6, 7.7, 7.8, 7.9, 8.0, 8.1, 8.2, 8.3, 8.4, 8.5, 8.6, 8.7, 8.8, 8.9, 9.0, 9.1, 9.2, 9.3, 9.4, 9.5 or any range therein.

In other embodiments of the invention, the buffer in the digestion buffer is TrisCl, which may be in the buffer at a concentration of about, at least about, or at most about 5, 10, 15, 20, 25, 30, 35, 40, 45, 50, 55, 60, 65, 70, 75, 80, 85, 90, 95, 100, 105, 110, 115, 120, 125, 130, 135, 140, 145, 150, 155, 160, 165, 170, 175, 180, 185, 190, 195, 200, 205, 210, 215, 220, 225, 230, 235, 240, 245, 250, 255, 260, 270, 275, 280, 285, 290, 295, 300, 310, 320, 330, 340, 350, 360, 370, 380, 390, 400, 410, 420, 430, 440, 450, 460, 470, 480, 490, 500 mM or any range therein. Although it is contemplated that other buffers may be employed as well.

In further embodiments, the digestion buffer contains a detergent. The detergent, particularly a mild one that is nondenaturing, can act to solubilize the sample. Detergents may be ionic or nonionic. The ionic detergent sodium dodecyl sulfate (SDS) is specifically contemplated for use in solutions of the invention. The concentration of the detergent in the buffer may be about, at least about, or at most about 0.1%, 0.2%, 0.3%, 0.4%, 0.5%, 0.6%, 0.7%, 0.8%, 0.9%, 1.0%, 1.5%, 2.0%, 2.5%, 3.0%, 3.5%, 4.0%, 4.5%, 5.0%, 5.5%, 6.0%, 6.5%, 7.0%, 7.5%, 8.0%, 8.5%, 9.0%, 9.5%, 10.0% or any range therein. It is contemplated that the concentration of the detergent can be up to an amount that allows the detergent to be soluble in the buffer.

In a specific embodiment, the digestion buffer includes 2% SDS, 200 mM TrisCl, pH 7.5, 200 mM NaCl, and 10 mM NaCitrate with 500 µg/ml of proteinase K. In further embodiments, it is specifically contemplated that the digestion buffer and/or any other steps of the invention involves a denaturant such as guanidinium. In some embodiments, a digestion buffer includes a denaturant at a concentrations of about or at most about 0.1, 0.2, 0.3, 0.4, 0.5, 0.6, 0.7, 0.8, 0.9, 1.0, 1.1, 1.2, 1.3, 1.4, 1.5, 1.6, 1.7, 1.8, 1.9, 2.0, 2.1, 2.2, 2.3, 2.4, 2.5 M or more, or any range therein. Methods and compositions of the invention will also be understood to exclude compounds, or limit their amount, that result in fragmented or truncated RNA molecules.

Solution used with methods of the invention may be added in a concentrated form or they may be provided in kits in a concentrated form. The solutions may be 2x, 3x, 4x, 5x, 10x, or 20x.

In some embodiments, the invention concerns methods for obtaining RNA from a fixed tissue sample by (a) contacting the fixed tissue sample with a digestion buffer comprising a polyanion and a protease to produce a lysate; (b) extracting RNA from the lysate. In specific embodiments, the polyanion is a polycarboxylate, such as sodium citrate. Such methods can be used with a biological sample that has been fixed, which may or may not be embedded in a non-reacting substance such as paraffin. The term "contacting" will be understood to have its plain and ordinary meaning to refer to the coming together of the solution and the sample. It will further be understood to encompass the terms "incubating," "exposing," "immersing" and "mixing."

In some embodiments of the invention, the ratio of fixed tissue sample and digestion buffer is from about 1 gram of tissue/5 ml digestion buffer to about 1 gram of tissue/25 ml digestion buffer or from about 1 gram of tissue/10 ml digestion buffer to about 1 gram of tissue/20 ml digestion buffer. It is contemplated that the ratio of any biological sample from which RNA is to be extracted is about, at least about, or at most about 1 gram of tissue to about 1, 2, 3, 4, 5, 6, 7, 8, 9, 10, 11, 12, 13, 14, 15, 16, 17, 18, 19, 20, 21, 22, 23, 24, 25, 26, 27, 28, 29, 30 ml or more of digestion buffer, or any range therein.

The fixed tissue may have been fixed by any means and the sample may have been obtained from any biological source. The sample also may have been embedded in a non-reactive substance such as paraffin. The invention also includes eliminating the substance prior to generating a cell lysate. In some embodiments, paraffin is eliminated by contacting the sample with a solution comprising an organic solvent, which is well known to those of skill in the art.

The amount of time that a sample is contacted with digestion buffers of the invention can be for about 1 to about 6 hours or about 4 hours. It is contemplated that the amount of time may be about, at least about or at most about 5, 10, 15, 20, 25, 30, 35, 40, 45, 50, 55 minutes and/or 1, 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48 or more hours, or any range therein.

The sample and the digestion buffer may be contacted with each other at temperatures that include, or are at least or at most about 20, 25, 30, 35, 40, 45, 50, 55, 60, 65, 70, 75, 80, 85, 90 °C or any range therein. In some embodiments, the temperature is between about 40°C and about 55°C. Such temperatures may or may not be maintained during the entire incubation period.

After incubation with the digestion buffer, a lysate may undergo homogenization, such as by a physical or mechanical device.

Additional methods of the invention concern extracting RNA from the lysate using a solution comprising alcohol and/or a solution comprising a non-alcohol organic solvent, such as phenol and/or chloroform. An alcohol solution is contemplated to contain at least one alcohol. The alcohol solution can be about, be at least about, or be at most about 5, 10, 15, 20, 25, 30, 35, 40, 45, 50, 55, 60, 65, 70, 75, 80, 85, 90, 95, 100% alcohol, or any range therein. In certain embodiments, it is added to a lysate to make the lysate have a concentration of alcohol of about, at least about, or at most about 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, 50, 51, 52, 53, 54, 55, 56, 57, 58, 59, 60, 61, 62, 63, 64, 65, 66, 67, 68, 69, 70, 71, 72, 73, 74, 75, 76, 77, 78, 79, 80, 81, 82, 83, 84, 85, 86, 87, 88, 89, 90%, or any range therein. In specific embodiments, the amount of alcohol added to a lysate renders it with an alcohol concentration of about 33%. In other embodiments, the amount of alcohol added to a mixture containing the lysate renders the concentration of 55% alcohol in the mixture. Alcohols include, but are not limited to, ethanol, propanol, isopropanol, butanol, and methanol. Ethanol is specifically contemplated for use in aspects of the invention. Extracting RNA from the lysate involves precipitating the RNA with alcohol in some embodiments of the invention.

The non-alcohol organic solvent solution is understood to contain at least one non-alcohol organic solvent, though it may also contain an alcohol. The concentrations described above with respect to alcohol solutions are applicable to concentrations of solutions having non-alcohol organic solvents. In specific embodiments, equal amounts of 1) the lysate and 2) phenol and/or chloroform are mixed.

In some embodiments, after a lysate has been digested and/or homogenized, but prior to further isolation procedures, other compounds can be added. In particular embodiments, a salt is added to the lysate in addition to an alcohol. The salt may be any salt, though in certain embodiments, the salt is guanidinium or sodium, or a combination of both. The amount of salt added to the lysate mixture (prior to the addition of an alcohol) can render the concentration of one or more salts in the mixture to be about, at least about, or at most about 0.1, 0.2, 0.3, 0.4, 0.5, 0.6, 0.7, 0.8, 0.9, 1.0, 1.1, 1.2, 1.3, 1.4, 1.5, 1.6, 1.7, 1.8, 1.9, 2.0, 2.1, 2.2, 2.3, 2.4, 2.5, 2.6, 2.7, 2.8, 2.9, 3.0, 3.1, 3.2, 3.3, 3.4, 3.5 M or more, or any range therein. In certain embodiments, guanidinium is added to the lysate to provide a concentration of guanidinium between about 0.5 and about 3 M. Consequently, the amount of guanidinium added to the lysate after homogenization provides a concentration of guanidinium that is about, at least about, or at most about 0.5, 0.6, 0.7, 0.8, 0.9, 1.0, 1.1, 1.2, 1.3, 1.4, 1.5, 1.6, 1.7, 1.8, 1.9, 2.0, 2.1, 2.2, 2.3, 2.4, 2.5, 2.6, 2.7, 2.8, 2.9, 3.0 M, or any range derivable therein. In other embodiments, a sodium salt such as sodium acetate or sodium chloride is also added to the lysate after homogenization to provide a concentration of this salt that is about, at least about, or at most about 0.1, 0.15, 0.2, 0.25, 0.3, 0.35, 0.4, 0.45, 0.5 M, or any range derivable therein. In certain embodiments, the following is added to the lysate prior to further isolation procedures: 1 volume of 4M guanidinium, 0.2 volumes NaAcetate at pH 4, then 2.75 volumes (1.25 of the final volume) ethanol.

Extraction of RNA from the lysate may further include using a mineral support. In some methods of the invention, a lysate that may or may not have been mixed with an alcohol or non-alcohol organic solvent solution is applied to a mineral support and the RNA is eluted from the support. Mineral supports include supports involving silica. In some embodiments, the silica is glass. Supports include, but are not limited to, columns and filters. In further embodiments, the mineral support is a glass fiber filter or column.

Alternatively, in some embodiments, extraction of RNA from the lysate can include a non-silica support. The support may include non-reactive materials, that is, materials that do not react chemically with the RNA to be isolated or extracted. Such materials include polymers or nonpolymers with electronegative groups. In some embodiments, the material is or has polyacrylate, polyacrylonitrile, polyvinylchloride, methacrylate, and/or methyl methacrylate.

Thus, some methods of the invention include (c) adding an alcohol solution to the lysate; (d) applying the lysate to a mineral support; and, (e) eluting the RNA from the mineral support with an elution solution. The mineral support may be washed 1, 2, 3, 4, 5 or more times after applying the lysate. Wash solutions include, in some embodiments, an alcohol, and in some cases, it also includes a salt. In further embodiments, the solution contains an alcohol concentration of about or at least about 50, 55, 60, 65, 70, 75, 80, 85, 90, 95%. In specific embodiments, the alcohol is ethanol. In additional embodiments, the salt concentration in the wash solution is about or is at least about 0.1, 0.2, 0.3, 0.4, 0.5, 0.6, 0.7, 0.8, 0.9, 1.0, 1.1, 1.2, 1.3, 1.4, 1.5, 1.6, 1.7, 1.8, 1.9, 2.0 M or more, or any range therein. Washes can be performed at a temperature that is about or is at least about 15, 20, 25, 30, 35, 40, 45, 50, 55, 60, 65, 70, 75, 80, 85, 90, 95, 100, 105, 110, 115, or 120°C, or any range derivable therein, including at an ambient temperature.

RNA can be eluted from a mineral support with an elution solution. In some embodiments, the elution solution includes EDTA. The concentration of EDTA in an elution solution is between about 0.01 mM to about 1.0 mM or between about 0.05 mM and about 0.5 mM. In specific embodiments, the concentration of EDTA in the elution solution is about 0.1 mM. The elution solution and/or the mineral support when elution solution is applied may be at room temperature or it may be heated to a temperature between about 80°C and about 100°C. In some embodiments, the temperature is about or is at least about 15, 20, 25, 30, 35, 40, 45, 50, 55, 60, 65, 70, 75, 80, 85, 90, 95, 100, 105, 110, 115, or 120°C, or any range derivable therein.

After RNA is extracted, individual or specific RNA molecules and/or pools of RNA molecules (as well as the entire population of isolated RNA) can be subject to additional reactions and/or assays. In some cases, these reactions and/or assays involve amplification of the RNA or of a DNA molecule generated from the RNA. For example, RT-PCR may be employed to generate molecules that can be characterized.

In some embodiments, a particular RNA molecule or an RNA population may be quantified, particularly the full-length RNA. Quantification includes any procedure known to those of skill in the art such as those involving one or more amplification reactions or RNase protection assays. These procedures include quantitative reverse transcriptase-PCR (qRT-PCR). In some embodiments, characterization of the isolated RNA is performed cDNA molecules are generated from the extracted RNA. Other characterization and quantification assays are contemplated as part of the invention. The methods and compositions of the invention allow full-length RNA to be quantified and characterized.

The RNAs can also be used in arrays or to generate cDNAs for use in arrays. Other assays include the use of spectrophotometry, electrophoresis, and sequencing.

The invention also includes kits for implementing the methods discussed above and/or kits that contain compositions discussed above. In some embodiments, kits of the invention include one or more of the following (consistent with compositions discussed above): a digestion buffer with a polycarboxylate and a protease; a glass fiber filter or column; elution buffer; wash buffer; alcohol solution; RNase inhibitor; and cDNA construction reagents (such as reverse transcriptase); reagents for amplification of RNA.

Any embodiments discussed with respect to compositions and/or methods of the invention, as well as any embodiments in the Examples, is specifically contemplated as being part of a kit.

It is contemplated that any embodiment of any method or composition described herein can be implemented with respect to any other embodiment of any method or composition described herein.

The use of the term "or" in the claims is used to mean "and/or" unless explicitly indicated to refer to alternatives only or the alternatives are mutually exclusive, although the disclosure supports a definition that refers to only alternatives and "and/or."

Throughout this application, the term "about" is used to indicate that a value includes the standard deviation of error for the device or method being employed to determine the value.

Following long-standing patent law, the words "a" and "an," when used in conjunction with the word "comprising" in the claims or specification, denotes one or more, unless specifically noted.

Other objects, features and advantages of the present invention will become apparent from the following detailed description. It should be understood, however, that the detailed description and the specific examples, while indicating specific embodiments of the invention, are given by way of illustration only, since various changes and modifications within the scope of the invention will become apparent to those skilled in the art from this detailed description.

### BRIEF DESCRIPTION OF THE DRAWINGS

The following drawings form part of the present specification and are included to further demonstrate certain aspects of the present invention. The invention may be better understood by reference to one or more of these drawings in combination with the detailed description of specific embodiments presented herein.
**FIG. 1****.** Shows the slow increase in mass as estimated using two different electrophoresis procedures - an agarose gel system (OD) and a capillary electrophoresis system (Aglient).
**FIG. 2****.** Samples subjected to qRT-PCR for GAPDH, FAS, Recc1, and DDPK did not show a drop in the cycle thresholds. The cycle thresholds are plotted against the time of digestion for each sample.
**FIG. 3****.** Bioanalyzer electropherograms show the comparsion of the procedure, which performs the proteolytic digestion in the presence of Na-citrate and terminates with a solid-phase extraction step with the precise procedure described in Masuda *et al.* (1999) for mouse liver that had been fixed for 14 weeks.
**FIG. 4****.** Effects of citrate versus Mg⁺⁺ at different temperatures.
**FIG. 5****.** Effects of digesting at different temperatures.
**FIG. 6****.** Effect of NaCl on digestion. All samples were quantified by OD₂₆₀ estimation to deduce the mass yield of RNA per gram of tissue.
**FIG. 7****.** Effects on the quality of RNA obtained are shown for 0.2 and 0.4 M NaCl at 2, 3 and 4 hr timepoints for levels of Recc1 and FAS RNA as determined by qRT-PCR.
**FIG. 8****.** The resultant RNA samples were analyzed on an Agilent Bioanalyzer 2100 RNA Chip and the percentage of 28 rRNA was determined. The 'Y-bar' column shows the average of the four livers and the 'S' column shows the standard deviation. Optimal citrate concentration under these conditions was at ∼50 mM (log = 1.7) while the optimal concentration under these conditions of SDS was at ∼3.5%.

### DESCRIPTION OF ILLUSTRATIVE EMBODIMENTS

The present invention overcome the deficiencies of current procedures of RNA isolation as is known in the art, by providing an optimized procedure that uses proteinase K in the presence of a polycarboxylic acid to isolate RNA of high quality (such as full-length RNA) and yield from fixed tissue.

### I. Fixation of Tissues Samples Comprising RNA

Tissue samples as contemplated for the procedure of the present invention are fixed tissue samples. Fixatives that may be used may include but are not limited to precipitant or non-precipitant fixatives. Two commonly used fixing agents are formaldehyde and paraformaldehyde. However, other fixatives may be employed in fixing tissue samples these include but are not limited acetic acid, formalin, osmium tetroxide, potassium dichromate, chromium trioxide, ethanol, mercuric chloride, methanol, glutaraldehyde and picric acid. Examples of fixatives and uses thereof may be found in Sambrook *et al.* (2000); Maniatis *et al.* (1989); Ausubel *et al.,* (1994); Jones *et al.* (1981); U.S. Patents 5,260,048, 4,946,669, 5,196,182.

Tissue samples may be fixed for about 1 hour, about 2 hours about 4 hours, about 6 hours, about 8 hours, about 10 hours, about 12 hours, or more hours; or about 1 day, about 2 days, about 3 days, about 4 days, about 5 days, about 6 days, or about 1, about 2 weeks, about 3 weeks; or about 1 month, about 2 months about 4 months, about 6 months, about 8 months, about 10 months; or about 1 or more years.

Examples of fixed tissue samples include, but are not limited to, heart, brain, testis, lungs, skeletal muscle, and spleen, liver and kidney.

Furthermore, the fixed tissue may or may not be embedded in a non-reactive substance such as paraffin. Methods and compositions of the invention can be applied to any fixed cell or tissue sample, whether it has been embedded or not.

### II. Digestion Buffers

A digestion buffer is employed to break down components of a cell. It is contemplated in the present invention that fixed tissue samples may be digested prior to extraction and analysis of the RNA. To accomplish this, various digestion buffers may be employed, and a variety of components of various concentration and pHs may be used in a digestion buffer to produce a lysate.

Various bases used in making buffers such as a digestion buffer are well known in the art. A digestion buffer may comprise of a Tris, TrisHCl, Tris borate, Hepes, or phosphate-buffered base, but is not limited to such. Such bases may further comprise of concentrations of about 10, 15, 20, 30, 40, 50, 60, 70, 80, 90, 100, 110, 120, 130, 140, 150, 160, 170, 180, 190, 200, 210, 220, 230, 240, 250 mM or greater. Such bases may be of varying pHs.

The pH of the digestion buffer or components thereof may about 1, about 1.5, about 2, about 2.5, about 3, about 3.5, about 4, about 4.5, about 5, about 5.5, about 6, about 6.5, about 7, about 7.5 about 8, about 8.5 about 9, about 9.5 or greater.

Salts such as NaCl, LiCl, or KCl, but not limited to such, may be used in a digestion buffer. These salts may also be included in a digestion buffer at various concentration of at least 1, 2, 3, 4, 5, 6, 7, 8, 9, 10, 15, 20, 25, 30, 35, 40, 45, 50, 100, 150, 200, 250, 300, 350, 400, 450, 500, 550, 600, 650, 700, 750, 800, 850, 900, 950, 1000, 2000, 3000, 4000, 5000 mM or more or greater. In some instances a salt may not be used. For example, see Harlow and Lane (1988); Sambrook *et al.* (2000); Maniatis *et al.* (1989) for a list of appropriate buffers and methods of making a digestion buffer.

It is contemplated that various detergents may be employed in a digestion buffer for producing a lysate form a fixed tissue sample. Detergents may be ionic, which include anionic and cationic detergents, or nonionic. Examples of nonionic detergents include triton, such as the Triton X series (Triton X-100, Triton X-100R, Triton X-114, Triton X-450, Triton X-450R), octyl glucoside, polyoxyethylene(9)dodecyl ether, digitonin, IGEPAL CA630, n-octyl-beta-D-glucopyranoside (betaOG), n-dodecyl-beta, C12EO7, Tween 20, Tween 80, polidocanol, n-dodecyl beta-D-maltoside (DDM), NP-40, C12E8 (octaethylene glycol n-dodecyl monoether), hexaethyleneglycol mono-n-tetradecyl ether (C14E06), octyl-beta-thioglucopyranoside (octyl thioglucoside, OTG), Emulgen, and polyoxyethylene 10 lauryl ether (C12E10). Examples of ionic detergents (anionic or cationic) include deoxycholate, sodium dodecyl sulfate (SDS), N-lauryl sarcosine, and cetyltrimethylammoniumbromide (CTAB). In some embodiments, urea may be added with or without another detergent or surfactant in a digestion buffer. A detergent may be of various concentration such as at least, 0.05%, 0.1%, 0.2%, 0.5%, 1.0 %, 2.0%, 4.0%, 5.0%, 6.0% , 7.0%, 8.0%, 10%, 20% or greater. Examples of detergents that may be used in a digestion buffer may be found in Harlow and Lane (1988); Sambrook (2000); Maniatis *et al.* (1989), each incorporated herein by reference.

The digestion may further include polyanions, having multiple acid groups, to enhance the quality of the lysate. Such polyanions may include but are not limited to polycarboxylates, such as trans-aconitic acid; 1,2,4-butanetricarboxylic acid; 1,4-cyclohexanedicarboxylic acid; 1,2,3,4,5,6-cyclohexanehexacarboxylic acid; 1,3,5-cyclohexanetricarboxylic acid; isocitric acid; tricarballylic acid; succinic acid; and glutaric acid.

A digestion buffer may further comprise a protease or peptidase to lyse a cell in order to isolate nucleic acids in the cell. Proteases are either an exopeptidase, which cleaves off amino acids from the ends of the protein chain, or an endopeptidases, which cleave peptide bonds within the protein. Typically, proteases are further categorized by mechanism, such as serine proteases (*e.g.*, chymotrypsin, trypsin, elastase, subtilisin, and proteinase K); cysteine (thiol) proteases (*e.g.*, bromelain, papain, cathepsins, parasitic proteases, and bacterial virulence factors); aspartic proteases (*e.g.*, pepsin, cathepsins, renin, fungal and viral proteases); and metalloproteases (*e.g.*, thermolysin). Proteinase K is commercially available and readily used for nucleic acid isolation and extraction procedures, as it is understood to be a highly thermostable protease that has very little cleavage specificity. With commercially available preparations of proteinase K, the end concentration is typically in the range of 0.05 to 1.0 mg/ml. It is contemplated that the end concentration of proteinase K in the context of a sample to be lysed can be, be at most, or be at least about 0.01, 0.02, 0.03, 0.04, 0.05, 0.06, 0.07, 0.08, 0.09, 0.10, 0.11, 0.12, 0.13, 0.14, 0.15, 0.16, 0.17, 0.18, 0.19, 0.20, 0.25, 0.30, 0.35, 0.40, 0.45, 0.50, 0.55, 0.60, 0.65, 0.70, 0.75, 0.80, 0.85, 0.90, 0.95, 1.0, 1.05, 1.10, 1.15, 1.20, 1.25, 1.30, 1.35, 1.40, 1.45, 1.50, or more mg/ml.

### III. RNA Extraction Procedure

The present invention further provides a method of isolating RNA from a paraffin embedded tissue. Many methods to isolate total RNA are well know to those skilled in the art. See, for example, Chomczynski and Sacchi (1987). The method to accomplish this task may employ the use of the Trizol reagent (Gibco Life Technologies) to extract total RNA. The Trizol procedure involves homogenization of the sample in a blender followed by extraction with the phenol-based Trizol reagent. The RNA is then precipitated with isopropyl alcohol and washed with ethanol before being redissolved in RNAse-free water or 0.5% SDS.

Other methods that may be employed the use of products known in the art such as RNAzol (Gibco BRL), TriReagent^{™} (Molecular Science), Qiagen's RNEasy Total RNA Isolation kit (Qiagen), Quickprep^{™} Total RNA Extraction kit (Amersham Bioscience) or any other maunufacture protocol for isolation of RNA. Other methods of RNA extraction include but are not limited to, the guanidine thiocyanate and cesium trifluoroacetate (CSTFA)method, the guanidinium hydrochloride method, or the lithium chloride - SDS- urea method. See Sambrook *et al.* (2000); *Maniatis et al.* (1989); Ausubel *et al.* (1994), for example of methods of RNA extraction.

RNA may be extracted from a variety of fixed tissue samples. Such tissues samples may comprise tissue of the brain, head, neck, gastrointestinal tract, lung, liver, pancreas, breast, testis, uterus, bladder, kidney, heart but is not limited to such tissues.

Solids supports may also be used for extracting the RNA from a fixed tissue sample and for maintaining or storing the RNA extracted. Such solid supports may include but are not limited to, spin columns, spin filters, vials, test tubes, flasks, bottles, elution columns or devices, filtration columns or devices, syringes and/or other container means. Such supports may further include plastic or glass beads or polymers such as cellulose. For examples see Sambrook *et al.* (2000) or Maniatis *et al.* (1989).

### IV. Uses of RNA from Fixed Tissue Samples

### A. Quantitation of RNA from Fixed Tissue Samples

RNA obtained from fixed tissue samples may be analyzed or quantitated by various methods to ascertain that the full length product is obtained. Provided herein are methods of quantitating or analyzing RNA. General methods for quantitating or analyzing RNA may be found in Sambrook *et al.* (2000) or Maniatis *et al.* 1(989). Below are provides examples of for using RNA form fixed tissue samples, however, these examples and are not meant to be limiting.

### 1. Quantitative PCR

The present invention relies on quantitative PCR - more specifically, quantitative RT-PCR - to quantitate the RNA in a sample. The methods may be semi-quantitative or fully quantitative.

Two approaches, competitive quantitative PCR^{™} and real-time quantitative PCR^{™}, both estimate target gene concentration in a sample by comparison with standard curves constructed from amplifications of serial dilutions of standard RNA. However, they differ substantially in how these standard curves are generated. In competitive QPCR, an internal competitor RNA is added at a known concentration to both serially diluted standard samples and unknown (environmental) samples. After coamplification, ratios of the internal competitor and target PCR^{™} products are calculated for both standard dilutions and unknown samples, and a standard curve is constructed that plots competitor-target PCR^{™} product ratios against the initial RNA concentration of the standard dilutions. Given equal amplification efficiency of competitor and RNA, the concentration of the latter in environmental samples can be extrapolated from this standard curve.

In real-time QPCR, the accumulation of amplification product is measured continuously in both standard dilutions of RNA and samples containing unknown amounts of RNA. A standard curve is constructed by correlating initial template concentration in the standard samples with the number of PCR^{™} cycles (*Cₜ*) necessary to produce a specific threshold concentration of product. In the test samples, the target PCR^{™} product accumulation is measured after the same *Cₜ*, which allows interpolation of target RNA concentration from the standard curve. Although real-time QPCR permits more rapid and facile measurement of RNA during routine analyses, competitive QPCR remains an important alternative for quantification in environmental samples. The coamplification of a known amount of competitor RNA with target RNA is an intuitive way to correct for sample-to-sample variation of amplification efficiency due to the presence of inhibitory substrates and large amounts of background RNA that are obviously absent from the standard dilutions.

Another type of QPCR is applied quantitatively PCR^{™}. Often termed "relative quantitative PCR," this method determines the relative concentrations of specific nucleic acids. In the context of the present invention, RT-PCR is performed on RNA samples isolated from fixed tissue samples.

In PCR^{™}, the number of molecules of the amplified RNA increase by a factor approaching two with every cycle of the reaction until some reagent becomes limiting. Thereafter, the rate of amplification becomes increasingly diminished until there is no increase in the amplified target between cycles. If a graph is plotted in which the cycle number is on the X axis and the log of the concentration of the amplified RNA is on the Y axis, a curved line of characteristic shape is formed by connecting the plotted points. Beginning with the first cycle, the slope of the line is positive and constant. This is said to be the linear portion of the curve. After a reagent becomes limiting, the slope of the line begins to decrease and eventually becomes zero. At this point the concentration of the amplified RNA becomes asymptotic to some fixed value. This is said to be the plateau portion of the curve.

The concentration of the RNA in the linear portion of the PCR^{™} amplification is directly proportional to the starting concentration of the target before the reaction began. By determining the concentration of the amplified products of the RNA in PCR^{™} reactions that have completed the same number of cycles and are in their linear ranges, it is possible to determine the relative concentrations of the specific target sequence in the original RNA mixture. If the RNA mixtures are cDNAs synthesized from RNAs isolated from different tissues, the relative abundances of the specific mRNA from which the target sequence was derived can be determined for the respective tissues. This direct proportionality between the concentration of the PCR^{™} products and the relative RNA abundances is only true in the linear range of the PCR^{™} reaction.

The final concentration of the RNA in the plateau portion of the curve is determined by the availability of reagents in the reaction mix and is independent of the original concentration of target DNA. Therefore, the first condition that must be met before the relative abundances of a RNA species can be determined by RT-PCR for a collection of RNA populations is that the concentrations of the amplified PCR^{™} products must be sampled when the PCR^{™} reactions are in the linear portion of their curves.

The second condition that must be met for a quantitative RT-PCR experiment to successfully determine the relative abundances of a particular RNA species is that relative concentrations of the amplifiable cDNAs must be normalized to some independent standard. The goal of an RT-PCR experiment is to determine the abundance of a particular RNA species relative to the average abundance of all RNA species in the sample.

Most protocols for competitive PCR^{™} utilize internal PCR^{™} standards that are approximately as abundant as the target. These strategies are effective if the products of the PCR amplifications are sampled during their linear phases. If the products are sampled when the reactions are approaching the plateau phase, then the less abundant product becomes relatively over represented. Comparisons of relative abundances made for many different RNA samples, such as is the case when examining RNA samples for differential expression, become distorted in such a way as to make differences in relative abundances of RNAs appear less than they actually are. This is not a significant problem if the internal standard is much more abundant than the target. If the internal standard is more abundant than the target, then direct linear comparisons can be made between RNA samples.

The above discussion describes theoretical considerations for an RT-PCR assay for clinically derived materials. The problems inherent in clinical samples are that they are of variable quantity (making normalization problematic), and that they are of variable quality (necessitating the co-amplification of a reliable internal control, preferably of larger size than the target). Both of these problems are overcome if the RT-PCR is performed as a relative quantitative RT-PCR with an internal standard in which the internal standard is an amplifiable cDNA fragment that is larger than the target cDNA fragment and in which the abundance of the RNA encoding the internal standard is roughly 5-100 fold higher than the RNA encoding the target. This assay measures relative abundance, not absolute abundance of the respective RNA species.

Other studies may be performed using a more conventional relative quantitative RT-PCR assay with an external standard protocol. These assays sample the PCR^{™} products in the linear portion of their amplification curves. The number of PCR^{™} cycles that are optimal for sampling must be empirically determined for each target cDNA fragment. In addition, the reverse transcriptase products of each RNA population isolated from the various tissue samples must be carefully normalized for equal concentrations of amplifiable cDNAs. This consideration is very important since the assay measures absolute mRNA abundance. Absolute RNA abundance can be used as a measure of differential gene expression only in normalized samples. While empirical determination of the linear range of the amplification curve and normalization of cDNA preparations are tedious and time consuming processes, the resulting RT-PCR assays can be superior to those derived from the relative quantitative RT-PCR assay with an internal standard.

One reason for this advantage is that without the internal standard/competitor, all of the reagents can be converted into a single PCR^{™} product in the linear range of the amplification curve, thus increasing the sensitivity of the assay. Another reason is that with only one PCR product, display of the product on an electrophoretic gel or another display method becomes less complex, has less background and is easier to interpret.

### 2. Denaturing Agarose Gel Electrophoresis

RNA extracted from a fixed tissue sample may be quantitated by agarose gel electrophoresis using a denaturing gel system. A positive control should be included on the gel so that any unusual results can be attributed to a problem with the gel or a problem with the RNA under analysis. RNA molecular weight markers, an RNA sample known to be intact, or both, can be used for this purpose. It is also a good idea to include a sample of the starting RNA that was used in the enrichment procedure.

Ambion's NorthernMax^{™} reagents for Northern Blotting include everything needed for denaturing agarose gel electrophoresis. These products are optimized for ease of use, safety, and low background, and they include detailed instructions for use. An alternative to using the NorthernMax^{™} reagents is to use a procedure described in "Current Protocols in Molecular Biology", Section 4.9 (Ausubel *et al.,* 1994), hereby incorporated by reference. It is more difficult and time-consuming than the Northern-Max method, but it gives similar results.

### 3. Agilent 2100 Bioanalyzer

RNA extracted from a fixed tissue sample may also be analyzed by an electrophoretic procedure that employs a capillary electrophoresis system. In the present invention, the Caliper RNA 6000 LabChip Kit and the Agilent 2100 Bioanalayzer are used. This system performs best with RNA solutions at concentrations between 50 and 250 ng/µl. Loading 1 µl of a typical enriched RNA sample is usually adequate for good performance. Follow the instructions provided with the RNA 6000 LabChip Kit for RNA analysis.

### 4. Assessing RNA Yield by UV absorbance

The concentration and purity of RNA can be determined by diluting an aliquot of the preparation (usually a 1:50 to 1:100 dilution) in TE (10 mM Tris-HCl pH 8, 1 mM EDTA) or water, and reading the absorbance in a spectrophotometer at 260 nm and 280 nm.

An A₂₆₀ of 1 is equivalent to 40 µg RNA/ml. The concentration (µg/ml) of RNA is therefore calculated by multiplying the A₂₆₀ X dilution factor X 40 µg/ml. The following is a typical example:
The typical yield from 10 µg total RNA is 3 - 5 µg. If the sample is re-suspended in 25 µl, this means that the concentration will vary between 120 ng/µl and 200 ng/µl. One µl of the prep is diluted 1:50 into 49 µl of TE. The A₂₆₀ = 0.1. RNA concentration = 0.1 X 50 X 40 µg/ml = 200 µg/ml or 0.2 µg/µl. Since there are 24 µl of the prep remaining after using 1 µl to measure the concentration, the total amount of remaining RNA is 24 µl X 0.2 µg/µl = 4.8 µg.

### 5. Assessing RNA yield with RiboGreen^{®}

Fluorescence-based assays may also be employed for quantitation of RNA. For example, the Molecular Probes' RiboGreen^{®} fluorescence-based assay for RNA quantitation can be employed to measure RNA concentration. RiboGreen reagent exhibits >1000-fold fluorescence enhancement and high quantum yield (0.65) upon binding nucleic acids, with excitation and emission maxima near those of fluorescein. Unbound dye is essentially nonfluorescent and has a large extinction coefficient (67,000 cm-1 M-1). The RiboGreen assay allows detection of as little as 1.0 ng/ml RNA in a standard fluorometer, filter fluorometer, or fluorescence microplate reader-surpassing the sensitivity achieved with ethidium bromide by 200-fold. The linear quantitation range for RiboGreen reagent extends over three orders of magnitude in RNA concentration.

### B. Other Uses of RNA from Fixed Tissue Samples

RNA obtained from a fixed tissue may be analyzed using microarray technology. For example an arrays such as a gene array are solid supports upon which a collection of gene-specific probes has been spotted at defined locations. The probes localize complementary labeled targets from a nucleic acid sample, such as an RNA sample, population via hybridization. One of the most common uses for gene arrays is the comparison of the global expression patterns of an RNA population. Typically, RNA isolated from two or more tissue samples may be used. The RNAs are reverse transcribed using labeled nucleotides and target specific, oligodT, or random-sequence primers to create labeled cDNA populations. The cDNAs are denatured from the template RNA and hybridized to identical arrays. The hybridized signal on each array is detected and quantified. The signal emitting from each gene-specific spot is compared between the populations. Genes expressed at different levels in the samples generate different amounts of labeled cDNA and this results in spots on the array with different amounts of signal.

The direct conversion of RNA populations to labeled cDNAs is widely used because it is simple and largely unaffected by enzymatic bias. However, direct labeling requires large quantities of RNA to create enough labeled product for moderately rare targets to be detected by array analysis. Most array protocols recommend that 2.5 g of polyA or 50 g of total RNA be used for reverse transcription (Duggan 1999). For practitioners unable to isolate this much RNA from their samples, global amplification procedures have been used.

The most often cited of these global amplification schemes is antisense RNA (aRNA) amplification (U.S. Patents 5,514,545 and 5,545,522). Antisense RNA amplification involves reverse transcribing RNA samples with an oligo-dT primer that has a transcription promoter such as the T7 RNA polymerase consensus promoter sequence at its 5' end. First strand reverse transcription creates single-stranded cDNA. Following first strand cDNA synthesis, the template RNA that is hybridized to the cDNA is partially degraded creating RNA primers. The RNA primers are then extended to create double-stranded DNAs possessing transcription promoters. The population is transcribed with an appropriate RNA polymerase to create an RNA population possessing sequence from the cDNA. Because transcription results in tens to thousands ofRNAs being created from each DNA template, substantive amplification can be achieved. The RNAs can be labeled during transcription and used directly for array analysis, or unlabeled aRNA can be reverse transcribed with labeled dNTPs to create a cDNA population for array hybridization. In either case, the detection and analysis of labeled targets are well known in the art. Other methods of amplification that may be employed include, but are not limited to, polymerase chain reaction (referred to as PCR^{™}; see U.S. Patents 4,683,195, 4,683,202 and 4,800,159, and Innis et al., 1988); and ligase chain reaction ("LCR"), disclosed in European Application No. 320 308, U.S. Patent 4,883,750, 5,912,148. Qbeta Replicase, described in PCT Application No. PCT/US87/00880, may also be used as an amplification method Alternative methods for amplification of a nucleic acid such as RNA are disclosed in U.S. Patents 5,843,650, 5,846,709, 5,846,783, 5,849,546, 5,849,497, 5,849,547, 5,858,652, 5,866,366, 5,916,776, 5,922,574, 5,928,905, 5,928,906, 5,932,451, 5,935,825, 5,939,291, 5,916,779 and 5,942,391, GB Application No. 2 202 328, and in PCT Application No. PCT/US89/01025, PCT Application WO 89/06700, PCT Application WO 88/10315, European Application No. 329 822, Kwoh et al., 1989; Frohman, 1994; Ohara *et al.,* 1989; and Walker *et al.*

cDNA libraries may also be constructed and used to analyze to RNA extracted from a fixed tissue sample. Construction of such libraries and analysis of RNA using such libraries may be found in Sambrook *et al.* (2000); Maniatis *et al.* (1989); Efstratiadis *et al.* (1976); Higuchi *et al.* (1976); Maniatis *et al.* (1976); Land *et al.* (1981); Okayama *et al.* (1982); Gubler *et al.* (1983); Ko (1990); Patanjali *et al.* (1991).

The present methods and kits may be employed for high volume screening. A library of RNA or DNA can be created using methods and compositions of the invention. This library may then be used in high throughput assays, including microarrays. Specifically contemplated by the present inventors are chip-based nucleic acid technologies such as those described by Hacia *et al.* (1996) and Shoemaker *et al.* (1996). Briefly, these techniques involve quantitative methods for analyzing large numbers of genes rapidly and accurately. By using fixed probe arrays, one can employ chip technology to segregate target molecules as high density arrays and screen these molecules on the basis of hybridization (see also, Pease *et al.,* 1994; and Fodor *et al,* 1991). The term "array" as used herein refers to a systematic arrangement of nucleic acid. For example, a nucleic acid population that is representative of a desired source (e.g., human adult brain) is divided up into the minimum number of pools in which a desired screening procedure can be utilized to detect or deplete a target gene and which can be distributed into a single multi-well plate. Arrays may be of an aqueous suspension of a nucleic acid population obtainable from a desired mRNA source, comprising: a multi-well plate containing a plurality of individual wells, each individual well containing an aqueous suspension of a different content of a nucleic acid population. Examples of arrays, their uses, and implementation of them can be found in U.S. Patent Nos. 6,329,209, 6,329,140, 6,324,479, 6,322,971, 6,316,193, 6,309,823, 5,412,087, 5,445,934, and 5,744,305.

Microarrays are known in the art and consist of a surface to which probes that correspond in sequence to gene products (*e.g*., cDNAs, mRNAs, cRNAs, polypeptides, and fragments thereof), can be specifically hybridized or bound at a known position. In one embodiment, the microarray is an array (*i.e.,* a matrix) in which each position represents a discrete binding site for a product encoded by a gene (e.g., a protein or RNA), and in which binding sites are present for products of most or almost all of the genes in the organism's genome. In a preferred embodiment, the "binding site" (hereinafter, "site") is a nucleic acid or nucleic acid analogue to which a particular cognate cDNA can specifically hybridize. The nucleic acid or analogue of the binding site can be, *e.g.*, a synthetic oligomer, a full-length cDNA, a less-than full length cDNA, or a gene fragment.

The nucleic acid or analogue are attached to a solid support, which may be made from glass, plastic (*e.g.*, polypropylene, nylon), polyacrylamide, nitrocellulose, or other materials. A preferred method for attaching the nucleic acids to a surface is by printing on glass plates, as is described generally by Schena *et al.,* 1995a. *See also* DeRisi et *al.,* 1996; Shalon *et al.,* 1996; Schena *et al.,* 1995b. Other methods for making microarrays, *e.g.*, by masking (Maskos *et al.,* 1992), may also be used. In principal, any type of array, for example, dot blots on a nylon hybridization membrane (see Sambrook et al., 1989, which is incorporated in its entirety for all purposes), could be used, although, as will be recognized by those of skill in the art, very small arrays will be preferred because hybridization volumes will be smaller.

Use of a biochip is also contemplated, which involves the hybridization of a labeled molecule or pool of molecules to the targets immobilized on the biochip.

### V. Kits

In further embodiments of the invention, there is a provided a kit for the isolation of full-length RNA from a fixed tissue sample. Any of the compositions described herein may be comprised in a kit. In a non-limiting example, reagents for fixing tissue samples, digesting and extracting RNA from the fixed tissue sample, and for analyzing or quantitating the RNA obtained may be included in a kit. The kits will thus comprise, in suitable container means, any of the reagents disclosed herein. It may also include one or more buffers, such as digestion buffer or a extracting buffer, and components for isolating the resultant RNA. Reagents for fixing tissue and reagents for embedding tissue may also be comprise in a kit.

The components of the kits may be packaged either in aqueous media or in lyophilized form. The container means of the kits will generally include at least one vial, test tube, flask, bottle, syringe or other container means, into which a component may be placed, and preferably, suitably aliquoted. Where there are more than one component in the kit (they may be packaged together), the kit also will generally contain a second, third or other additional container into which the additional components may be separately placed. However, various combinations of components may be comprised in a vial. The kits of the present invention also will typically include a means for containing the RNA, and any other reagent containers in close confinement for commercial sale. Such containers may include injection or blow-molded plastic containers into which the desired vials are retained. When the components of the kit are provided in one and/or more liquid solutions, the liquid solution is an aqueous solution, with a sterile aqueous solution being particularly preferred.

However, the components of the kit may be provided as dried powder(s). When reagents and/or components are provided as a dry powder, the powder can be reconstituted by the addition of a suitable solvent. It is envisioned that the solvent may also be provided in another container means. The container means will generally include at least one vial, test tube, flask, bottle, syringe and/or other container means, into which the nucleic acid formulations are placed, preferably, suitably allocated. The kits may also comprise a second container means for containing a sterile, pharmaceutically acceptable buffer and/or other diluent.

Such kits may also include components that facilitate isolation of the extracted RNA. It may also include components that preserve or maintain the RNA or that protect against its degradation. Such components may be RNAse-free or protect against RNAses. Such kits generally will comprise, in suitable means, distinct containers for each individual reagent or solution.

A kit will also include instructions for employing the kit components as well the use of any other reagent not included in the kit. Instructions may include variations that can be implemented.

### VI. EXAMPLES

The following examples are included to demonstrate preferred embodiments of the invention. It should be appreciated by those of skill in the art that the techniques disclosed in the examples which follow represent techniques discovered by the inventor to function well in the practice of the invention, and thus can be considered to constitute preferred modes for its practice. However, those of skill in the art should, in light of the present disclosure, appreciate that many changes can be made in the specific embodiments which are disclosed and still obtain a like or similar result without departing from the scope of the invention.

### EXAMPLE 1

### PROCEDURES

### Fixation of Tissue Containing RNA

Two fixing agents have been tested, formaldehyde and paraformaldehyde. Paraformaldehyde was used at 4% final concentration in phosphate-buffered saline solution (PBS, 50 mM Na-phosphate buffer, pH 7.5, 150 mM NaCl), and formaldehyde was used in a 3.7% final concentration in similar buffer ("10% Neutral Buffered Formalin", NBF, Protocol(TM) Formalin (cat#305-510) from Fisher Diagnostics, Middletown, VA). For either fixative, the fixation procedure used entailed soaking a sample of tissue excised from a freshly-sacrificed mouse at 0-8°C for the span of time specified. The time of fixation is specified in each example. Samples were either stored in fixative or transferred to paraffin blocks after passage through several changes of ethanol and xylene in the standard method. The tissue sample was dehydrated in the following solutions:
i) 70% ethanol 1h,
ii). 80% ethanol 1h,
iii). 90% ethanol 1h,
iv). 100% ethanol (I) 2h,
v). 100% ethanol (II) 2 h,
vi). 100% ethanol (III) overnight.
This procedure was followed by the step of clearing and embedding of the tissue in the following solutions:
i). Ethanol/Xylene (1:1) 1h
ii). Xylene 2h,
iii). Xylene 2h,
iv). Xylene/Paraffin (1:1; 55-60°C) 2h,
v). Paraffin; 55-60°C 2h,
vi). Paraffin; 55-60°C 2h,
vii). Paraffin; 55-60°C for 1hr.
The final step involved the embedding of the block with paraffin in a paraffin mold. This allowed for the samples to be stable for at least a half year.

### RNA Extraction

For extraction from paraffin, the tissue was soaked in two changes of xylene for 5 minutes at 50°C after which the sample is placed in digestion buffer. For tissue still stored in fixative, the sample was soaked in a series of ethanol solutions as follows: the tissue was placed into 2-5ml of 30% EtOH pre-chilled on ice (larger tissues require the 5 ml) and incubated on ice for 10 min then transferred to the same volume of 40% EtOH pre-chilled on ice and incubate on ice for an additional 10 min. This process was repeated with 10% increasing increments of EtOH until reaching 100%. The tissue was soaked in 100% EtOH at least overnight at 4°C. In the final step, the tissue was removed from the solution (EtOH, Formalin, or Xylene) and dried on a paper towel. The tissue sample was then transferred to digestion buffer containing 200 mM TrisCl, pH 7.5, 200 mM NaCl, 10 mM NaCitrate, 2% SDS, and 0.5 mg/ml PK.

The ratio of tissue to digestion buffer (in g/ml) fell in the range 1:10-20. The sample was homogenized until the sample was fully dispersed, usually 10-30s. This was performed most conveniently with a rotor-stator homogeizer at high-speed. Samples were then incubated at 50-52°C for 4h, with a useful range of 1-6 hr, enabling to proteinase K to digest away most of the protein. After this incubation, the lysate was made 33% in ethanol by the addition of one-half volume of 100% EtOH. Alternatively, after the incubation, guanidinium and a sodium salt, such as sodium acetate (pH 4), were added to the lysate to yield a concentration of about 2 M guanidinium (400 µl of 4 M GuSCN added to 400 µl lysate) and between about 0.1 and 0.2 M sodium acetate (80 µl of 2 M sodium acetate, pH 4 was added to a 400 µl lysate); thereafter, ethanol was added (1.1 ml ethanol) to yield a solution that is 55% ethanol prior to applying the lysate mixture to the glass fiber filter.

The sample was mixed and then applied to an RNAqueous glass fiber filter and spun at max (13,200 rpm) 1 min at room temperature (RT). The flowthrough was discarded. 700 µl Wash Solution 1 (1.6 M Guanadinium Thiocyanate, 0.2% N-Lauryl Sarcosine, 10 mM Sodium Citrate, 40 mM 2-Mercapethanol, 0.3 M Sodium Acetate pH 7.2) was applied to filter followed by centrifugation at max (13,200 rpm) 1 min at room temperature (RT). 500 µl Wash Solution 2/3 (80% ethanol, 0.1 M NaCl, 4.5 mMM EDTA, 10 mM Tris pH 7.5) was applied to filter and centrifuged at max (13,200 rpm) 1 min at room temperature (RT). 500 µl Wash Solution 2/3 is applied to filter and centrifuged at max (13,200 rpm) 1 min at room temperature (RT). The flowthrough from each was discarded and the filter containing the sample was spun at max speed for 1 min. The filter was then transferred to a new collection tube. 30 µl of 0.1 mM EDTA heated to 95-100 °C was applied to the filter. The sample was spun at max speed for 30s at RT. This elution (30 µl hot 0.1 mM EDTA solution applied and spun through at maximum centrifugation speed for 30s) was repeated and the eluate either analyzed immediately or stored at -20°C until analyzed. Analysis was performed as described in the following sections. No differences were apparent with extended storage at -20°C.

### EXAMPLE 2

### ANALYSIS OF RNA BY ELECTROPHORESIS

RNA samples were examined by two different electrophoretic procedures. The first was an agarose gel system (NorthernMax Gly, Ambion) which provided both an ethidium-stained pattern and the ability to create Northern Blots to probe for the presence of discreet bands for specific mRNAs (specifically, GAPDH and β-actin). The second was a capillary electrophoresis system (the RNA chip, Caliper Technologies Corp., used on the 2100 Bioanalyzer, Agilent). This analysis was applied with respect to the Examples discussed below.

### EXAMPLE 3

### ANALYSIS of RNA by QUANTITATIVE RT-PCR (REAL-TIME or QRT-PCR)

The presence of specific mRNAs were quantified through the use of quantitative or real-time RT-PCR (Higuchi *et al.,* 1993; Bustin, 2000). By monitoring the presence of PCR products initially templated on specific mRNAs during the actual amplification reaction, the relative levels of these specific mRNAs in different samples were ascertained. For the present studies, four mRNAs were targeted, GAPDH, Recc1, FAS, and DDPK. For each, the following RT-PCR primers and probes were used.

GAPDH - Mus musculus glyceraldehyde-3-phosphate dehydrogenase (Gapd), mRNA. Accession# - NM_008084; Amplicon - 60nt; mRNA size - 1.23kb; Target region in the mRNA: 396-455; Probe 415-434 [5'FAM- TGCCGATGCCCCCATGTTTG -3'TAMRA] (SEQ ID NO:1); Primers - Forward: 5'TCATCATCTCCGCCCCTT (SEQ ID NO:2) and Reverse: 5' TCTCGTGGTTCACACCCATC (SEQ ID NO:3). Recc1 - Mus musculus replication factor C (Recc1), mRNA; Accession# - NM_011258; Amplicon - 83nt; mRNA size - 4.68kb; Target region in the mRNA: 2122-2204; Probe - 2156 - 2176 [5'FAM-CCTTCCGTGAGTGCGAGGCAC-3'TAMRA] (SEQ ID NO:4); Primers - Forward: 5'CAGCATCAAAGGCTTTTATACAAGTG (SEQ ID NO:5) and Reverse: 5' TGCCATCGACCTCATCCA (SEQ ID NO:6). FAS - Mus musculus fatty acid synthase (Fasn), mRNA; Accession# - NM_007988; Amplicon - 122nt; mRNA Size - 8.36kb; Target region in the mRNA: 6857 - 6978; Probe - 6915 - 6937 [5'FAM-CCTGAGGGACCCTACCGCATAGC-3'TAMRA] (SEQ ID NO:7); Primers - Forward: 5'CCTGGATAGCATTCCGAACCT (SEQ ID NO:8) and Reverse: 5'AGCACATCTCGAAGGCTACACA (SEQ ID NO:9). DDPK - Mus musculus protein kinase, DNA activated, catalytic polypeptide (Prkdc), mRNA; Accession# - NM_011159; Amplicon - 127nt; mRNA Size - 12.65kb; Target region in the mRNA: 3101- 3227; Probe - 3157 - 3179 [5'FAM-AGCAAGTCACTTTTCAAGCGGCT-3'TAMRA] (SEQ ID NO:10; Primers - Forward: 5'TCAAATGGTCCATTAAGCAA.ACAA (SEQ ID NO:11) and Reverse: 5'GCTGCACCTAGCCTCTTGAAA (SEQ ID NO:12).

To compare samples, 10 µl of the RNA sample (prepared from equivalent amounts of tissue) was combined with 2 µl Random Decamers (from the RetroScript kit, Ambion) and incubated at 80°C for 3 min. After this incubation, 8 µl of a master RT mixture (per 8 µl: 2 µl 10X RT Buffer, 4 µl dNTP mix (2.5 mM each), 1 µl (10 U) RNase Inhibitor (RIP), and 1 µl (100 U) Moloney Murine Leukemia Virus Reverse Transcriptase (MMLV-RT; all from RetroScript kit, Ambion)) was added, and incubated at 50°C for one hour to transcribe all RNA into cDNA. Prior to qPCR, MMLV-RT is deactivated by incubation at 92°C for 10 min or stored at -20°C

For qPCR, 2 µl of this cDNA mixture was added to 18 µl of a PCR Master Mix using components provided in the SuperTaq RealTime^{™} kit (Ambion) as well as primers and probe for the specific mRNA to be quantified [per 18 µl, 2 µl 10X RealTime Buffer, 2 µl dNTP mix (2.5 mM ea), 3 µl 25 mM MgCl₂, 0.4 µl 50X ROX, 0.2 µl SuperTaq (1U), 0.4 µl Probe (5 µM, 100 nM final), 1µl of the specific Primer set (10 µM each, 500 nM each final), and 9 µl water]. This was then monitored during PCR using an ABI 7000 real-time machine and the following thermal cycling conditions: 10 min at 95 °C; then 15 sec at 95 °C followed by 60 sec at 60 °C for 40 cycles.

### EXAMPLE 4

### TIME-COURSE of DIGESTION of FIXED MOUSE LIVER

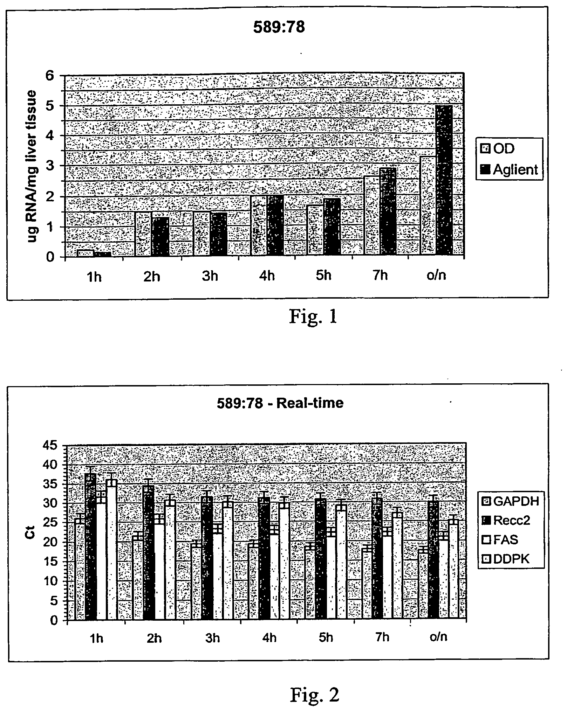

Digestion was performed on a mouse liver sample that had been fixed 27 days. The digestion was performed according to standard conditions at 50°C, with 200 µl aliquots removed at various times and extracted for RNA as described previously. Equivalent amounts of the RNA samples from each time-point were then analyzed by electrophoresis on the Agilent Bioanalyzer 2100 RNA chip and quantified spectrophotometrically. The 2100 RNA chip data allowed quantification of total RNA mass, which was compared with that calculated from the OD₂₆₀ of each sample. FIG. 1 shows the slow increase in mass as estimated by both these procedures. The mass of the samples rose rapidly until 2 hr, then increased much more slowly from 3-5 hr. Both the 7 hr and overnight (o/n) digestion times seem to provide an extra amount of RNA. However, when equivalent amounts of these samples (representing increasing mass inputs) were subjected to qRT-PCR for GAPDH, FAS, Recc1, and DDPK, the cycle thresholds did not drop accordingly, as shown in FIG. 2, where the cycle thresholds are plotted against the time of digestion for each sample. It is apparent that, during the course of digestion, only incremental increases in the yield of viable template mRNA are obtained after 3 hr. During this plateau phase, it was also noted that the ΔCₜ between genes stays relatively constant, indicating that time of digestion had little effect on the populational representation of various genes.

### EXAMPLE 5

### COMPARISON of PROCEDURES

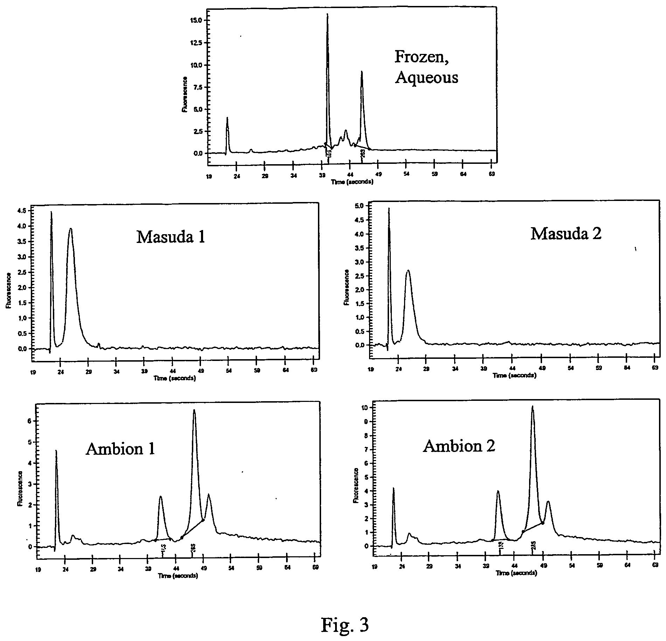

Masuda *et al.* (1999) reported being able to obtain full-length RNA (as judged from the presence of rRNA bands on an electrophoretic gel) from fixed samples using their procedure, which contained a proteinase K digestion solution containing MgCl₂ and terminated using organic extractions and ethanol precipitation. Thus, the procedure of the present invention, which performs the proteolytic digestion in the presence of Na-citrate and terminates with a solid-phase extraction step as described above, was compared with the precise procedure described in Masuda *et al*. (1999) for mouse liver that had been fixed for 14 weeks. The tissue was split and homogenized in the digestion solution and digestion conditions followed as specified by each procedure - 1 hr at 45°C for the Masuda procedure, and 4 hr at 50°C for the present invention. After digestion, the RNA was extracted by organic or solid-phase extraction as specified by each procedure, and the ethanol pellet from the Masuda preparation was redissolved in the same volume (60 µl) as that used to elute in the procedure of the present invention. For each sample, duplicates were examined on ethidium-stained gels and by the Agilent Bioanalyzer 2100 RNA chip electrophoretic methods, using equal volume amounts (from equivalent masses of tissue). The Bioanalyzer electropherograms are shown in FIG. 3. A profile of RNA from a non-fixed sample of tissue obtained by the RNAqueous method is shown as well, to provide a reference. The presence of material in the upper molecular-weight range is apparent in both the non-fixed and samples of the present invention, and it can be seen that there are peaks in the samples that are similar to the rRNA peaks seen in the standard profile.

In addition to this direct qualitative assessment, the Agilent 2100 RNA chip method was also used for quantification of total RNA mass, which was compared with that calculated from the OD₂₆₀ of each sample. From these methods, the yield from the Masuda protocol was 0.187±0.027 µg RNA/mg tissue by OD, and 0.083±0.025 by Bioanalyzer, while the yield from the method of the present invention was 0.183±0.064 and 0.191±0.047 by the same methods. The smaller Bioanalyzer number from the Masuda protocol indicated that there is a great deal more fragmentation, with mono- and dinucleotides not visible in the Bioanalyzer analysis. This was further supported by analyzing the level of FAS by qRT-PCR. By this method, the cycle threshold for the Masuda samples was 35.1±2.4, but only 26.8±2.1 for the samples of the invention, indicating an approximately 2^(35.1-26.8)=315-fold greater amount of amplifiable FAS mRNA in the sample of the present invention.

Moreover, the Masuda protocol provides 0.101 µg of RNA per mg of tissue, with a peak size of 131 nt and only 1% of area under the curve in the size range greater than 350 nt. This is to be contrasted with the prep from unfixed ("frozen") tissue, which, although it has a similar yield (0.153 µg/mg tissue), shows two peaks at approximately 1830 and 3130 nt, representing the 18S and 28S rRNAs (actual size for the 28S should be approximately 5000 nt), and has 86% of its area under the curve in the region larger than 350 nt. The samples using our technique also give a similar yield (0.191 +/- 0.047 µg/mg tissue), but shows 79.1 +/- 1.3% of its area larger than 350 nt, and shows peaks at ∼2060 and ∼3390, a reasonable size for modified rRNAs (the small peak is even larger).

### EXAMPLE 6

### EFFECTS of CITRATE VERSUS MG⁺⁺ at DIFFERENT TEMPERATURES

Two digestion buffers with identical components (200 mM TrisCl, pH 7.5; 200 mM NaCl; 2% SDS; 0.5 mg/ml PK) were made that had in addition either MgCl₂ at 1.5 mM or NaCitrate at 10 mM. Separate pieces of the same fixed mouse liver were put into either of these buffers and homogenized, then each was split three ways and incubated at three different temperatures, 42°C, 50°C, and 65°C, for 4 hr. After the digestion step, RNA was extracted by the solid-phase method described above.

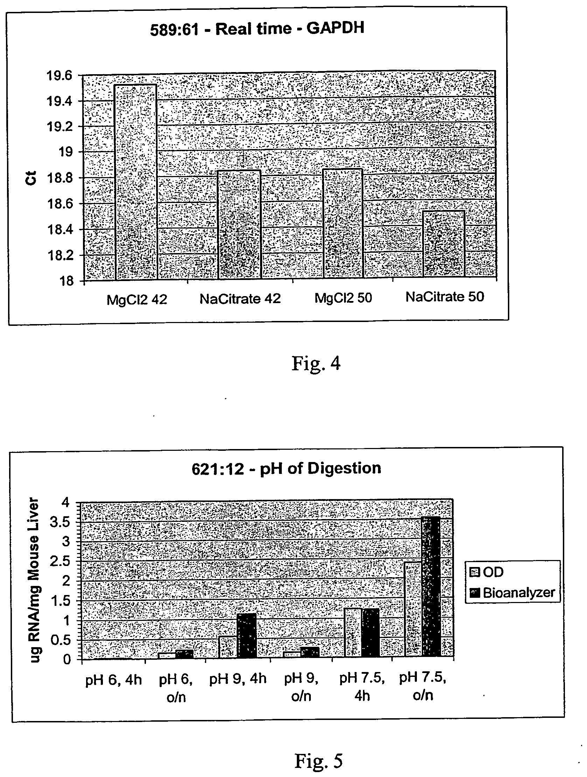

Samples were examined by electrophoresis on both agarose gels and the Bioanalyzer 2100. The quantification from the bioanalyzer and the absorbance at 260 nm indicated that the samples were roughly equivalent, having about 0.8 mg RNA per gram tissue by OD₂₆₀ and about half that by Bioanalyzer. The appearance was variable, with the samples incubated at 65°C apparently degraded. Equivalent amounts of the 42°C and 50°C samples were subjected to qRT-PCR for GAPDH mRNA and at either temperature, the NaCitrate-incubated samples had more viable mRNA present (lower Cₜ, FIG. 4).

### EXAMPLE 7

### EFFECTS OF DIGESTING AT DIFFERENTpH's

Two additional digestion buffers were made, with all components but TrisCl the same as in the standard buffer (200 mM TrisCl, pH 7.5; 200 mM NaCl; 10 mM NaCitrate, 2% SDS; 0.5 mg/ml PK). These two had the 200 mM TrisCl at pH 7.5 substituted with 200 mM TrisCl at pH 9.0 or 200 mM MES(Na⁺) at pH 6.0, providing alternative digestion buffers at pH 6, 7.5, or 9. Three pieces of the same fixed mouse kidney sample were divided and homogenized into each of these three digestion solutions, then incubated at 50°C. Samples were removed at 4 hr and after overnight incubation, and RNA was extracted over glass fiber filters as described. The samples were examined by agarose gel, Bioanalyzer 2100 RNA chip and OD₂₆₀ estimation of mass yield. The pH 6 samples quickly degraded, but the pH 7.5 and 9 samples were comparable at the 4 hr incubation, although the pH 9 was visibly more degraded after the overnight incubation. The mass yield from the OD₂₆₀ and Agilent Bioanalyzer data also indicated that pH 7.5 was optimal (FIG. 5)

### EXAMPLE 8

### EFFECT of NaCl on DIGESTION

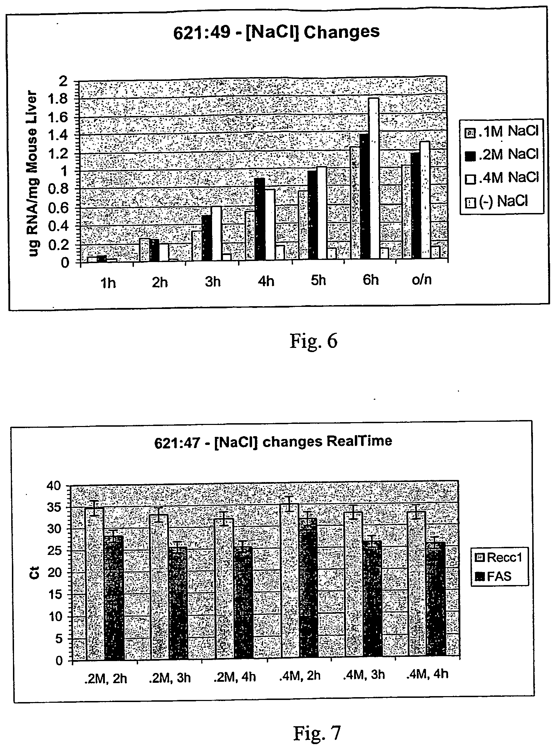

Three additional digestion buffers were made, with all components but NaCl the same as in the standard buffer (200 mM TrisCl, pH 7.5; 200 mM NaCl; 10 mM NaCitrate, 2% SDS; 0.5 mg/ml PK). The additional buffers had the 200 mM NaCl substituted with 100 mM, 400 mM, or no salt. Four pieces of a fixed mouse liver sample were homogenized separately in each of these digestion buffers (at the same final tissue:buffer ratio), and the samples were incubated at 50 °C. At specific times, equal aliquots of each digest were removed and RNA was extracted over glass fiber filters as described. The samples were examined by agarose gel electrophoresis and the quality of RNA did not seem to vary significantly between samples, although the zero-salt sample showed no visible product. All the samples were quantified by OD₂₆₀ estimation, to deduce the mass yield of RNA per gram of tissue. This is shown in FIG. 6, and indicates that some NaCl is essential for good extraction of RNA, although differences between the yields for any of the salt-containing buffers is minimal. To look at possible effects on the quality of RNA obtained, the two best concentration, 0.2 M and 0.4 M NaCl, were selected, and the 2, 3, and 4 hr timepoints looked at for levels of Recc1 and FAS RNAs as determined by qRT-PCR as described above. This data is shown in FIG. 7 and shows little effect between these two concentrations, although 0.2 M may be slightly advantageous.

### EXAMPLE 9

### ALTERNATIVES TO CITRATE

NaCitrate is the sodium salt of the tricarboxylic acid, citric acid. Other commonly used reagents with the presence of multiple acid groups were also tested to see if they could also provide this enhancement. Nine compounds were chosen: trans-aconitic acid; 1,2,4-butanetricarboxylic acid; 1,4-cyclohexanedicarboxylic acid; 1,2,3,4,5,6-cyclohexanehexacarboxylic acid; 1,3,5- cyclohexanetricarboxylic acid; isocitric acid; tricarballylic acid; succinic acid; and glutaric acid. A digestion solution was made without NaCitrate (200 mM TrisCl, pH 7.5; 200 mM NaCl; 2% SDS; 0.5 mg/ml PK), and a piece of fixed mouse liver was homogenized in this solution. The homogenate was then parsed into 10 aliquots and 1/10^{th} volume of each had added a 1 M stock of the polyacid to be tested. All samples were incubated at 50°C for 4 hr, then prepped for RNA as in the normal procedure. Each of these samples was examined for both yield and appearance on the Bioanalyzer 2100. The following table gives an appraisal of their appearance relative to the citrate sample, as well as the mass yield for each.

| **Compound** | **Appearance** | **Yield (µg RNA/mg mouse liver)** |
|---|---|---|
| Na Citrate | 0 | 0.749 |
| trans-aconitic acid | - - | 0.384 |
| 1,2,4-butanetricarboxylic acid | - - - | 0.232 |
| 1,4-cyclohexanedicarboxylic acid | + | 1.24 |
| 1,2,3,4,5,6-cyclohexanehexacarboxylic acid | - - - | 0.152 |
| 1,3,5-cyclohexanetricarboxylic acid | - | 0.358 |
| Isocitric acid | + | 1.08 |
| Tricarballylic acid | - - - | 0.179 |
| Succinic acid | 0 | 0.762 |
| Glutaric acid | - - | 0.613 |
| (0=comparable to NaCitrate; +=superior to NaCitrate; - to ---=progressively inferior to NaCitrate) | | |

The compounds 1,4-cyclohexanedicarboxylic acid and isocitric acid both appear to function well in this procedure, and other polycarboxylates would be expected to as well.

### EXAMPLE 10

### PARAFFIN EMBEDDED TISSUE

Samples of mouse liver and kidney tissue that had been fixed for 3 months were passed into paraffin blocks as described above. These samples were split with a razor and homogenized in digestion solution three different ways: by direct homogenization of the paraffin-encrusted piece; by homogenization of a piece that had been de-paraffinized with two 5 min soaks of xylene at 50°C; and by de-paraffinization with a 20 min xylene soak at 50°C followed by a transition into ethanol with three 3 min soaks in absolute ethanol. All three procedures provided RNA amenable to analysis for each tissue. Fixed samples of mouse heart, brain, testes, lungs, skeletal muscle, and spleen have been successfully used in this procedure as well as liver and kidney as demonstrated in the above examples.

### EXAMPLE 11

### EFFECTS OF CITRATE CONCENTRATIONS AND SDS

Four (4) mouse livers were harvested, fixed for 24 hr in NBF, and embedded in paraffin as per standard protocols. After one week in paraffin, each liver was shaved of excess paraffin, then thoroughly crushed under liquid nitrogen. The paraffin/tissue powder was put through two xylene soaks and two ethanol soaks to thoroughly remove paraffin, then the second ethanol slurry was distributed to nine tubes, at ∼100 mg tissue per tube, where the tissue was pelleted and excess ethanol removed prior to adding digestion media. Digestion was with 0.5 mg/ml Proteinase K in each of the buffers above plus 200 mM TrisCl at pH 7.5, for 3 hr at 50°C.

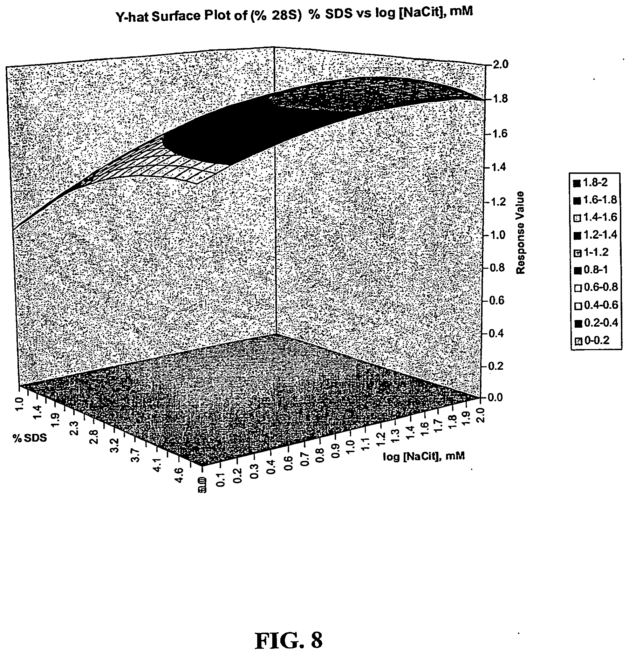

After digestion, an equal volume of 4 M guanidine thiocyanate (GuSCN) and one-tenth volume of 2 M Na-acetate, pH 4 were added prior to mixing with 1.25 volumes of ethanol. This was applied to a glass fiber filter device (from Ambion RNAqueous^{®} kit), washed once with 1.7 M GuSCN/70% ethanol, then with Washes 2 and 3 and eluted as described in the RNAqueous^{®} protocol and in Example 1 above. The resultant RNA samples were analyzed on an Agilent Bioanalyzer 2100 RNA Chip and the percentage of 28 rRNA was determined (FIG. 8). The 'Y-bar' column shows the average of the four livers and the 'S' column shows the standard deviation. Optimal citrate concentration under these conditions was at ∼50 mM (log = 1.7) while the optimal concentration under these conditions of SDS was at ∼3.5%.

| Factorset # | % SDS | log [NaCit], mM | Ybar | S |
|---|---|---|---|---|
| 1 | 1 | 0 | 1.0225 | 0.115578 |
| 2 | 1 | 1 | 1.365 | 0.165227 |
| 3 | 1 | 2 | 1.6425 | 0.170563 |
| 4 | 3 | 0 | 1.365 | 0.257488 |
| 5 | 3 | 1 | 1.855 | 0.235301 |
| 6 | 3 | 2 | 1.8175 | 0.163987 |
| 7 | 5 | 0 | 1.5475 | 0.098107 |
| 8 | 5 | 1 | 1.745 | 0.081035 |
| 9 | 5 | 2 | 1.8125 | 0.292504 |

All of the compositions and/or methods and/or apparatus disclosed and claimed herein can be made and executed without undue experimentation in light of the present disclosure. While the compositions and methods of this invention have been described in terms of preferred embodiments, it will be apparent to those of skill in the art that variations may be applied to the compositions and/or methods and/or apparatus and in the steps or in the sequence of steps of the method described herein without departing from the concept, spirit and scope of the invention. More specifically, it will be apparent that certain agents that are both chemically and physiologically related may be substituted for the agents described herein while the same or similar results would be achieved. All such similar substitutes and modifications apparent to those skilled in the art are deemed to be within the scope and concept of the invention as defined by the appended claims.

### REFERENCES

U.S. Patent 4,683,195
U.S. Patent 4,683,202
U.S. Patent 4,800,159
U.S. Patent 4,883,750
U.S. Patent 4,946,669
U.S. Patent 5,196,182
U.S. Patent 5,260,048
U.S. Patent 5,514,545
U.S. Patent 5,545,522
U.S. Patent 5,843,650
U.S. Patent 5,846,709
U.S. Patent 5,846,783
U.S. Patent 5,849,497
U.S. Patent 5,849,546
U.S. Patent 5,849,547
U.S. Patent 5,858,652
U.S. Patent 5,866,366
U.S. Patent 5,912,148
U.S. Patent 5,916,776
U.S. Patent 5,916,779
U.S. Patent 5,922,574
U.S. Patent 5,928,905
U.S. Patent 5,928,906
U.S. Patent 5,932,451
U.S. Patent 5,935,825
U.S. Patent 5,939,291
U.S. Patent 5,942,391
U.S. Patent 6,284,535
U.S. Patent 6,428,963
Abrahamsen et al., J. Mol. Diagn., 5(1):34-41, 2003.
Ausubel et al., In: Current Protocols in Molecular Biology, John, Wiley & Sons, Inc, New York, 1994.
Bock et al., Anal Biochem., 295(1):116-7, 2001.
Bustin, J. Mol. Endocrinol., 25(2):169-193, 2000.
Chomczynski and Sacchi, Anal. Biochem., 162(1):156-159, 1987.
Coombs et al., Nucleic Acids Res., 27(16):E12, 1999.
Efstratiadis et al., Cell, 7:279-3680, 1976.
European Appln. 320 308
European Appln. 329 822
Fang et al., Biotechniques, 33(3):604, 606, 608-610, 2002.
Frohman, In: PCR Protocols: A Guide To Methods And Applications, Academic Press, N.Y., 1994.
GB Appln. 2 202 328
Godfrey et al., J. Mol. Diagn., 2(2):84-91, 2000.
Gubler et al., Gene, 25:263-269, 1983.
Harlow and Lane, Antibodies: A Laboratory Manual. Cold Spring Harbor Laboratory Press, Cold Spring harbor, NY, 553-612, 1988.
Higuchi et al., Biotechnology, 11(9):1026-1030, 1993.
Higuchi et al., Proc. Natl. Acad. Sci. USA, 73:3146-3150, 1976.
Innis et al., Proc. Natl. Acad. Sci. USA, 85(24):9436-9440, 1988.
Jones, et al., Laboratory Investigations 44:32A, 1981.
Karsten et al., Nucleic Acids Res., 30(2):E4, 2002.
Ko, Nucleic Acids Res. ,18:5705-5711, 1990.
Koopmans et al., J. Virol. Methods, 43(2):189-204, 1993.
Krafft et al., Mol. Diagn., 2(3):217-230, 1997.
Kwoh et al., Proc. Natl. Acad. Sci. USA, 86:1173, 1989.
Land et al., Nucleic Acids Res., 9:2251-2266, 1981.
Liu et al., Diagn. Mol. Patrol., 11(4):222-227,2002.
Maniatis et al., Cell, 8:163-182, 1976.
Maniatis, et al., Molecular Cloning, A Laboratory Manual, Cold Spring Harbor Press, Cold Spring Harbor, N.Y., 1988.
Masuda et al., Nucleic Acids Res., 27(22):4436-4443, 1999.
Ohara et al., Proc. Natl. Acad. Sci. USA, 86:5673-5677, 1989.
Okayama et al., Mol. Cell. Biol., 2:161-170, 1982.
Patanjali et al., Proc. Natl. Acad. Sci. USA, 88:1943-1947, 1991.
PCT Application PCT/US87/00880
PCT Application PCT/US89/01
PCT Application WO 88/10315
PCT Application WO 89/06700
Sambrook et al., In: Molecular Cloning: A Laboratory Manual, second edition, Cold Spring Harbor Laboratory, Cold Spring Harbor, NY, 2000.
Specht et al., Am. J. Pathol., 158(2):419-429, 2001.
Van Deerlin et al., Neurochem. Res., 27(10):993-1003, 2002. Walker et al., Proc. Natl. Acad. Sci. USA, 89:392-396 1992.

## Claims

1. A method for isolating RNA from a fixed tissue sample comprising:
(a) contacting the fixed tissue sample with a digestion buffer, comprising a polyanion, a protease, and optionally guanidinium, to produce a lysate, wherein if guanidinium is present in the digestion buffer it is present at a concentration of at most 1.8 M;
(b) extracting RNA from the lysate,
wherein the polyanion is a polycarboxylate, selected from the group consisting of sodium citrate, 1,4-cyclohexanedicarboxylic acid, 1,3,5-cyclohexanetricarboxylic acid, isocitric acid, and succinic acid, preferably sodium citrate.

2. The method of claim 1, wherein the digestion buffer comprises up to about 5% SDS, about 200 mM TrisCl, pH 7.5, about 200 mM NaCl, and up to about 100 mM sodium citrate with about 500 µg/ml of proteinase K.

3. The method of claim 1, wherein the concentration in the digestion buffer of the polyanion is between about 1 mM and about 100 mM, preferably about 50 mM.

4. The method of claim 1, wherein the digestion buffer further comprises sodium.

5. The method of claim 1, wherein the protease in the digestion buffer is proteinase K.

6. The method of claim 1, wherein the pH of the digestion buffer is between about 7.0 and about 9.5.

7. The method of claim 1, wherein the ratio of fixed tissue sample and digestion buffer is from about 1 gram of tissue/5 ml digestion buffer to about 1 gram of tissue/25 ml digestion buffer.

8. The method of claim 7, wherein the ratio of fixed tissue sample and digestion buffer is from about 1 gram of tissue/10 ml digestion buffer to about 1 gram of tissue/20 ml digestion buffer.

9. The method of claim 1, wherein the fixed tissue sample is contacted with the digestion buffer for about 1 to about 6 hours, preferably for about 4 hours.

10. The method of claim 1, wherein the temperature of the contacting is between about 40 C and about 55 C.

11. The method of claim 1, wherein extracting RNA from the lysate comprises:
(b1) adding an alcohol solution to the lysate;
(b2) applying the lysate to a mineral support; and,
(b3) eluting the RNA from the mineral support with an elution solution.

12. The method of claim 11, further comprising washing the mineral support after the lysate has been applied.

13. The method of claim 11, wherein the mineral support is a glass fiber filter or column.

14. The method of claim 11, wherein the elution solution comprises EDTA, preferably in a concentration of about 0.01 mM to about 1.0 mM.

15. The method of claim 11, wherein the elution solution is at a temperature between about 80 C and about 100 C.

16. The method of claim 11, further comprising adding one or more salts to the lysate in step (b1), preferably prior to the addition of the alcohol solution.

17. The method of claim 16, wherein guanidinium is a salt added to the lysate, preferably yielding a concentration of the guanidinium in the lysate between about 0.5 M and about 3.0 M.

18. The method of claim 17, wherein a sodium salt is also added to the lysate.

19. The method of claim 1, wherein the RNA is extracted from the lysate using a solution comprising a non-alcohol organic solvent, preferably phenol.

20. The method of claim 1, further comprising quantifying an amount of RNA extracted from the lysate, preferably by using an amplification reaction.

21. The method of claim 1, further comprising generating cDNA molecules from the extracted RNA.

22. The method of claim 1, wherein the fixed tissue sample is embedded in paraffin.

23. The method of claim 22, further comprising eliminating paraffin from the sample, preferably by contacting the embedded sample with an organic solvent.

24. A kit for isolating full-length RNA from a fixed tissue sample comprising:
(a) a digestion buffer to produce a lysate, the digestion buffer comprising a polycarboxylate, a protease, and guanidinium, wherein the concentration of the guanidinium is between 0.1-1.8 M; and,
(b) a glass fiber filter or column,
wherein the polycarboxylate is selected from the group consisting of sodium citrate, 1,4-cyclohexanedicarboxylic acid, 1,3,5-cyclohexanetricarboxylic acid, isocitric acid, and succinic acid, preferably sodium citrate.

25. The kit of claim 24, wherein the digestion buffer comprises: up to about 5% SDS, about 200 mM TrisCl, pH 7.5, about 200 mM NaCl, up to about 100 mM sodium citrate, about 500 µg/ml of proteinase K, and 0.1-1.8 M guanidinium.

## Patentansprüche

1. Verfahren zum Isolieren von RNA aus einer fixierten Gewebeprobe, umfassend:
(a) Inberührungbringen der fixierten Gewebeprobe mit einem Verdaupuffer, umfassend ein Polyanion, eine Protease und optional Guanidinium, um ein Lysat herzustellen, wobei, falls Guanidinium in dem Verdaupuffer vorhanden ist, es in einer Konzentration von höchstens 1,8 M vorhanden ist;
(b) Extrahieren von RNA aus dem Lysat,
wobei das Polyanion ein Polycarboxylat, das aus der Gruppe ausgewählt ist, bestehend aus Natriumcitrat, 1,4-Cyclohexandicarbonsäure, 1,3,5-Cyclohexantricarbonsäure, Isocitronensäure und Bernsteinsäure, vorzugsweise Natriumcitrat, ist.

2. Verfahren nach Anspruch 1, wobei der Verdaupuffer bis zu etwa 5 % SDS, etwa 200 mM TrisCl, einen pH-Wert von 7,5, etwa 200 mM NaCl und bis zu etwa 100 mM Natriumcitrat mit etwa 500 µg/ml Proteinase K umfasst.

3. Verfahren nach Anspruch 1, wobei die Konzentration des Polyanions in dem Verdaupuffer zwischen etwa 1 mM und etwa 100 mM, vorzugsweise etwa 50 mM, beträgt.

4. Verfahren nach Anspruch 1, wobei der Verdaupuffer ferner Natrium umfasst.

5. Verfahren nach Anspruch 1, wobei die Protease in dem Verdaupuffer Proteinase K ist.

6. Verfahren nach Anspruch 1, wobei der pH-Wert des Verdaupuffers zwischen etwa 7,0 und etwa 9,5 beträgt.

7. Verfahren nach Anspruch 1, wobei das Verhältnis von fixierter Gewebeprobe und Verdaupuffer von etwa 1 Gramm Gewebe/5 ml Verdaupuffer bis etwa 1 Gramm Gewebe/25 ml Verdaupuffer beträgt.

8. Verfahren nach Anspruch 7, wobei das Verhältnis von fixierter Gewebeprobe und Verdaupuffer von etwa 1 Gramm Gewebe/10 ml Verdaupuffer bis etwa 1 Gramm Gewebe/20 ml Verdaupuffer beträgt.

9. Verfahren nach Anspruch 1, wobei die fixierte Gewebeprobe mit dem Verdaupuffer für etwa 1 bis etwa 6 Stunden, vorzugsweise für etwa 4 Stunden, in Berührung gebracht wird.

10. Verfahren nach Anspruch 1, wobei die Temperatur des Inberührungbringens zwischen etwa 40 C und etwa 55 C beträgt.

11. Verfahren nach Anspruch 1, wobei das Extrahieren von RNA aus dem Lysat umfasst:
(b1) Zusetzen einer Alkohollösung zu dem Lysat;
(b2) Aufbringen des Lysats auf einen mineralischen Träger; und,
(b3) Eluieren von RNA aus dem mineralischen Träger mit einer Elutionslösung.

12. Verfahren nach Anspruch 11, ferner umfassend ein Waschen des mineralischen Trägers, nachdem das Lysat aufgebracht wurde.

13. Verfahren nach Anspruch 11, wobei der mineralische Träger ein Glasfaserfilter oder eine Glasfasersäule ist.

14. Verfahren nach Anspruch 11, wobei die Elutionslösung EDTA, vorzugsweise in einer Konzentration von etwa 0,01 mM bis etwa 1,0 mM, umfasst.

15. Verfahren nach Anspruch 11, wobei die Elutionslösung auf einer Temperatur zwischen etwa 80 C und etwa 100 C ist.

16. Verfahren nach Anspruch 11, ferner umfassend das Zusetzen eines oder mehrerer Salze zu dem Lysat in Schritt (b1), vorzugsweise vor dem Zusatz der Alkohollösung.

17. Verfahren nach Anspruch 16, wobei Guanidinium ein Salz, das zu dem Lysat zugesetzt wird, ist, was vorzugsweise eine Konzentration des Guanidiniums in dem Lysat zwischen etwa 0,5 M und etwa 3,0 M ergibt.

18. Verfahren nach Anspruch 17, wobei zu dem Lysat ebenso ein Natriumsalz zugesetzt wird.

19. Verfahren nach Anspruch 1, wobei die RNA aus dem Lysat unter Verwendung einer Lösung, umfassend ein nicht alkoholisches organisches Lösungsmittel, vorzugsweise Phenol, extrahiert wird.

20. Verfahren nach Anspruch 1, ferner umfassend ein Quantifizieren einer RNA-Menge, die aus dem Lysat extrahiert wird, vorzugsweise durch Verwenden einer Amplifikationsreaktion.

21. Verfahren nach Anspruch 1, ferner umfassend ein Erzeugen von cDNA-Molekülen aus der extrahierten RNA.

22. Verfahren nach Anspruch 1, wobei die fixierte Gewebeprobe in Paraffin eingebettet ist.

23. Verfahren nach Anspruch 22, ferner umfassend ein Eliminieren von Paraffin aus der Probe, vorzugsweise durch Inberührungbringen der eingebetteten Probe mit einem organischen Lösungsmittel.

24. Kit zum Isolieren von RNA voller Länge aus einer fixierten Gewebeprobe, umfassend:
(a) einen Verdaupuffer, um ein Lysat herzustellen, wobei der Verdaupuffer ein Polycarboxylat, eine Protease und Guanidinium umfasst, wobei die Konzentration des Guanidiniums zwischen 0,1-1,8 M beträgt; und,
(b) einen Glasfaserfilter oder eine Glasfasersäule,
wobei das Polycarboxylat aus der Gruppe ausgewählt ist, bestehend aus Natriumcitrat, 1,4-Cyclohexandicarbonsäure, 1,3,5-Cyclohexantricarbonsäure, Isocitronensäure und Bernsteinsäure, vorzugsweise Natriumcitrat.

25. Kit nach Anspruch 24, wobei der Verdaupuffer umfasst: bis zu etwa 5 % SDS, etwa 200 mM TrisCl, einen pH-Wert von 7,5, etwa 200 mM NaCl, bis zu etwa 100 mM Natriumcitrat, etwa 500 µg/ml Proteinase K und 0,1-1,8 M Guanidinium.

## Revendications

1. Procédé permettant d'isoler de l'ARN à partir d'un échantillon de tissu fixé comprenant :
(a) la mise en contact de l'échantillon de tissu fixé avec un tampon de digestion, comprenant un polyanion, une protéase et facultativement du guanidinium, pour produire un lysat, dans lequel si le guanidinium est présent dans le tampon de digestion, il est présent à une concentration d'au plus 1,8 M ;
(b) l'extraction de l'ARN du lysat,
dans lequel le polyanion est un polycarboxylate, choisi dans le groupe constitué par le citrate de sodium, l'acide 1,4-cyclohexanedicarboxylique, l'acide 1,3,5-cyclohexanetricarboxylique, l'acide isocitrique et l'acide succinique, de préférence le citrate de sodium.

2. Procédé selon la revendication 1, dans lequel le tampon de digestion comprend jusqu'à environ 5 % de SDS, environ 200 mM de TrisCl, pH 7,5, environ 200 mM de NaCl, et jusqu'à environ 100 mM de citrate de sodium avec environ 500 µg/ml de protéinase K.

3. Procédé selon la revendication 1, dans lequel la concentration dans le tampon de digestion du polyanion est comprise entre environ 1 mM et environ 100 mM, de préférence environ 50 mM.

4. Procédé selon la revendication 1, dans lequel le tampon de digestion comprend en outre du sodium.

5. Procédé selon la revendication 1, dans lequel la protéase dans le tampon de digestion est la protéinase K.

6. Procédé selon la revendication 1, dans lequel le pH du tampon de digestion est compris entre environ 7,0 et environ 9,5.

7. Procédé selon la revendication 1, dans lequel le rapport de l'échantillon de tissu fixé et du tampon de digestion va d'environ 1 gramme de tissu/5 ml de tampon de digestion à environ 1 gramme de tissu/25 ml de tampon de digestion.

8. Procédé selon la revendication 7, dans lequel le rapport de l'échantillon de tissu fixé et du tampon de digestion va d'environ 1 gramme de tissu/10 ml de tampon de digestion à environ 1 gramme de tissu/20 ml de tampon de digestion.

9. Procédé selon la revendication 1, dans lequel l'échantillon de tissu fixé est mis en contact avec le tampon de digestion pendant environ 1 à environ 6 heures, de préférence pendant environ 4 heures.

10. Procédé selon la revendication 1, dans lequel la température de la mise en contact est comprise entre environ 40 °C et environ 55 °C.

11. Procédé selon la revendication 1, dans lequel l'extraction d'ARN du lysat comprend :
(b1) l'ajout d'une solution alcoolique au lysat ;
(b2) l'application du lysat sur un support minéral ; et,
(b3) l'élution de l'ARN du support minéral avec une solution d'élution.

12. Procédé selon la revendication 11, comprenant en outre le lavage du support minéral après l'application du lysat.

13. Procédé selon la revendication 11, dans lequel le support minéral est un filtre ou une colonne en fibre de verre.

14. Procédé selon la revendication 11, dans lequel la solution d'élution comprend de l'EDTA, de préférence en une concentration d'environ 0,01 mM à environ 1,0 mM.

15. Procédé selon la revendication 11, dans lequel la solution d'élution se trouve à une température comprise entre environ 80 °C et environ 100 °C.

16. Procédé selon la revendication 11, comprenant en outre l'ajout d'un ou plusieurs sels au lysat à l'étape (b1), de préférence avant l'ajout de la solution alcoolique.

17. Procédé selon la revendication 16, dans lequel le guanidinium est un sel ajouté au lysat, produisant de préférence une concentration du guanidinium dans le lysat comprise entre environ 0,5 M et environ 3,0 M.

18. Procédé selon la revendication 17, dans lequel un sel de sodium est également ajouté au lysat.

19. Procédé selon la revendication 1, dans lequel l'ARN est extrait du lysat à l'aide d'une solution comprenant un solvant organique non alcoolique, de préférence du phénol.

20. Procédé selon la revendication 1, comprenant en outre la quantification d'une quantité d'ARN extrait du lysat, de préférence à l'aide d'une réaction d'amplification.

21. Procédé selon la revendication 1, comprenant en outre la génération de molécules d'ADNc à partir de l'ARN extrait.

22. Procédé selon la revendication 1, dans lequel l'échantillon de tissu fixé est incorporé dans de la paraffine.

23. Procédé selon la revendication 22, comprenant en outre l'élimination de la paraffine de l'échantillon, de préférence par mise en contact de l'échantillon incorporé avec un solvant organique.

24. Trousse permettant d'isoler de l'ARN pleine longueur à partir d'un échantillon de tissu fixé comprenant :
(a) un tampon de digestion pour produire un lysat, le tampon de digestion comprenant un polycarboxylate, une protéase, et du guanidinium, la concentration du guanidinium étant comprise entre 0,1 et 1,8 M ; et,
(b) un filtre ou une colonne en fibre de verre,
dans laquelle le polycarboxylate est choisi dans le groupe constitué par le citrate de sodium, l'acide 1,4-cyclohexanedicarboxylique, l'acide 1,3,5-cyclohexanetricarboxylique, l'acide isocitrique et l'acide succinique, de préférence le citrate de sodium.

25. Trousse selon la revendication 24, dans laquelle le tampon de digestion comprend :
jusqu'à environ 5 % de SDS, environ 200 mM de TrisCl, pH 7,5, environ 200 mM de NaCl,
jusqu'à environ 100 mM de citrate de sodium, environ 500 µg/ml de protéinase K, et 0,1 à 1,8 M de guanidinium.
